# EUROPEAN PATENT APPLICATION

(11) **EP 3 933 033 A1**
(43) Date of publication of application: **05.01.2022**
(21) Application number: 20763776.0
(22) Date of filing: 26.02.2020
(51) Int. Cl.: C12N 5/077, C12N 5/10, A61L 27/38

(54) **METHOD FOR PRODUCING OSTEOBLAST CLUSTER USING IPS CELLS**

(30) Priority: 26.02.2019 JP 2019033277
(71) Applicant: Tohoku University, Sendai-shi, Miyagi 980-8577 (JP)
(72) Inventor: EGUSA, Hiroshi, Sendai-shi, Miyagi 980-8577 (JP); OKAWA, Hiroko, Sendai-shi, Miyagi 980-8577 (JP); HORIE, Naohiro, Sendai-shi, Miyagi 980-8577 (JP); KONDO, Takeru, Sendai-shi, Miyagi 980-8577 (JP)
(74) Representative: Winter, Brandl - Partnerschaft mbB
(86) International application number: PCT/JP2020/007876
(87) International publication number: WO 2020/175592

(57) **Abstract**

Provided is a method of producing an osteoblast construct from iPS cells, the method including the steps of: (1) inducing formation of an embryoid body by subjecting undifferentiated iPS cells to non-adherent culture; (2) inducing differentiation of the iPS cells into mesodermal cells by subjecting the embryoid body of the iPS cells obtained in the step (1) to non-adherent culture; and (3) inducing differentiation into osteoblasts by subjecting the mesodermal cells of the iPS cells obtained in the step (2) to non-adherent culture, wherein the steps (1) and (2) are each performed using a culture vessel comprising a bottom surface and a circular side wall arranged upright on the bottom surface, the bottom surface having a plurality of depressed portions arranged independently of each other.

SEQUENCE LISTING

<110>  TOHOKU UNIVERSITY

<120>  Method for producing osteoblast culster using iPS Cells

<130>  P20-005WO

<150>  JP2019-033277
<151>  2019-02-26

<160>  28

<170>  PatentIn version 3.5

<210>  1
<211>  18
<212>  DNA
<213>  Artificial Sequence

<220>
<223>  primer

<400>  1
cgggctacct gccatcac                                                    18


<210>  2
<211>  20
<212>  DNA
<213>  Artificial Sequence

<220>
<223>  primer

<400>  2
ggccagaggc agaagtcaga                                                  20


<210>  3
<211>  21
<212>  DNA
<213>  Artificial Sequence

<220>
<223>  primer

<400>  3
ctcgtctgac tgcctgccta g                                                21


<210>  4
<211>  20
<212>  DNA
<213>  Artificial Sequence

<220>
<223>  primer

<400>  4
gcgtggatgc ctgccttgta                                                  20

<210>  5
<211>  19
<212>  DNA
<213>  Artificial Sequence

<220>
<223>  primer

<400>  5
tgtcccaacc cccaaagac                                                    19


<210>  6
<211>  23
<212>  DNA
<213>  Artificial Sequence

<220>
<223>  primer

<400>  6
ccctcgactc ctacatcttc tga                                               23


<210>  7
<211>  20
<212>  DNA
<213>  Artificial Sequence

<220>
<223>  primer

<400>  7
ccgggagcag tgtgagctta                                                   20


<210>  8
<211>  20
<212>  DNA
<213>  Artificial Sequence

<220>
<223>  primer

<400>  8
ccgggagcag tgtgagctta                                                   20


<210>  9
<211>  20
<212>  DNA
<213>  Artificial Sequence

<220>
<223>  primer

<400>  9
tctccttgcg ccacagaatg                                                   20


<210>  10
<211>  20
<212>  DNA

<213>  Artificial Sequence

<220>
<223>  primer

<400>  10
tccttagact caccgctctt                                                   20


<210>  11
<211>  20
<212>  DNA
<213>  Artificial Sequence

<220>
<223>  primer

<400>  11
cggaggagac aacggagaag                                                   20


<210>  12
<211>  20
<212>  DNA
<213>  Artificial Sequence

<220>
<223>  primer

<400>  12
gtaagtgtcg ccacgaggct                                                   20


<210>  13
<211>  20
<212>  DNA
<213>  Artificial Sequence

<220>
<223>  primer

<400>  13
gtaacccgtt gaaccccatt                                                   20


<210>  14
<211>  20
<212>  DNA
<213>  Artificial Sequence

<220>
<223>  primer

<400>  14
ccatccaatc ggtagtagcg                                                   20


<210>  15
<211>  20
<212>  DNA
<213>  Artificial Sequence

<220>

<223>  primer

<400>  15
cagaccagca gcactccata                                                    20


<210>  16
<211>  20
<212>  DNA
<213>  Artificial Sequence

<220>
<223>  primer

<400>  16
cagcgtcaac accatcattc                                                    20


<210>  17
<211>  20
<212>  DNA
<213>  Artificial Sequence

<220>
<223>  primer

<400>  17
aagctgatct ggtggtgcat                                                    20


<210>  18
<211>  20
<212>  DNA
<213>  Artificial Sequence

<220>
<223>  primer

<400>  18
gactccacaa agggcatgat                                                    20


<210>  19
<211>  20
<212>  DNA
<213>  Artificial Sequence

<220>
<223>  primer

<400>  19
gtgctaaagg tgccaatggt                                                    20


<210>  20
<211>  20
<212>  DNA
<213>  Artificial Sequence

<220>
<223>  primer

<400>  20

ctcctcgctt tccttcctct                                                    20


<210>  21
<211>  19
<212>  DNA
<213>  Artificial Sequence

<220>
<223>  primer

<400>  21
cactcctcgc cctattggc                                                     19


<210>  22
<211>  21
<212>  DNA
<213>  Artificial Sequence

<220>
<223>  primer

<400>  22
ccctcctgct tggacacaaa g                                                  21


<210>  23
<211>  20
<212>  DNA
<213>  Artificial Sequence

<220>
<223>  primer

<400>  23
aactggccga agaatagcaa                                                    20


<210>  24
<211>  20
<212>  DNA
<213>  Artificial Sequence

<220>
<223>  primer

<400>  24
tgcaccaggt ctgagtgttc                                                    20


<210>  25
<211>  20
<212>  DNA
<213>  Artificial Sequence

<220>
<223>  primer

<400>  25
cagtcagtac cccagcctgt                                                    20

<210>  26
<211>  20
<212>  DNA
<213>  Artificial Sequence

<220>
<223>  primer

<400>  26
actggctgtc cacgatgtct                                                   20


<210>  27
<211>  20
<212>  DNA
<213>  Artificial Sequence

<220>
<223>  primer

<400>  27
gaaggtgaag gtcggagtca                                                   20


<210>  28
<211>  20
<212>  DNA
<213>  Artificial Sequence

<220>
<223>  primer

<400>  28
gaagatggtg atgggatttc                                                   20

## Description

### Technical Field

### [Cross-reference to Related Application]

This application claims priority from Japanese Patent Application No. 2019-033277, filed on February 26, 2019, the entire disclosure of which is incorporated herein by reference. The present invention relates to a method of generating an osteoblast construct using iPS cells.

### Background Art

There is an extremely high demand for an artificial bone/bone substitute material for replacing, for example, a defective site where bone has been lost due to bone tumor excision, comminuted fracture, bone defect associated with fixation in rheumatoid arthritis, alveolar ridge resorption, or the like.

Non-absorptive materials, such as hydroxyapatite, and absorptive materials, such as β-tricalcium phosphate, which are currently in clinical use as artificial bones/bone substitute materials, have a problem of, for example, being inferior to autologous bones in osteoinductive activity, and do not always provide good prognosis in surgical intervention. In addition, a hybrid artificial bone/bone substitute material obtained by combining an artificial bone and a growth factor protein, such as a bone morphogenetic protein (BMP), which is a next-generation type whose further development is desired, lacks an "extracellular matrix", which is important for bone tissue regeneration, and hence has not been able to provide a sufficient bone regeneration effect.

Under such circumstances, one of the inventors of the present invention has succeeded in generating a bone regeneration agent including an inactivated cell construct derived from stem cells as a source material, the inactivated cell construct containing at least a mineral and an extracellular matrix (Patent Literatures 1 and 2).

### Citation List

### Patent Literature

PTL 1: WO 2015/064705 A1
PTL 2: WO 2018/181960 A1

### Non-patent Literature

NPL 1: Stem Cell Reports Vol. 2, 751-760, June 3, 2014

### Summary of Invention

### Technical Problem

An object of the present invention is to provide a novel method of producing an osteoblast construct, capable of providing an osteoblast construct having a high bone regeneration ability through use of iPS cells as a source material.

### Solution to Problem

Under the above-mentioned circumstances, the inventors of the present invention have made extensive investigations, and as a result, have surprisingly found that an osteoblast construct having an extremely high bone regeneration ability is obtained by utilizing microspace culture in each of (1) an embryoid body formation induction step and (2) a step of inducing differentiation into mesodermal cells in a method of producing an osteoblast construct using undifferentiated iPS cells as a source material, including the embryoid body formation induction step, the step of inducing differentiation into mesodermal cells, and a step of inducing differentiation into osteoblasts. The inventors of the present invention have further made minute investigations on culture conditions and the like on the basis of the novel finding, and thus have completed the present invention.

Accordingly, the present invention provides a method, an osteoblast construct, and the like as described in the following items.
Item 1. A method of producing an osteoblast construct from iPS cells, including the steps of:
   (1) inducing formation of an embryoid body by subjecting undifferentiated iPS cells to non-adherent culture;
   (2) inducing differentiation of the iPS cells into mesodermal cells by subjecting the embryoid body of the iPS cells obtained in the step (1) to non-adherent culture; and
   (3) inducing differentiation into osteoblasts by subjecting the mesodermal cells of the iPS cells obtained in the step (2) to non-adherent culture,
   wherein the steps (1) and (2) are each performed using a culture vessel comprising a bottom surface and a circular side wall arranged upright on the bottom surface, the bottom surface having a plurality of depressed portions arranged independently of each other.
Item 2. The method according to Item 1, wherein at least one of the plurality of depressed portions has a circle-equivalent diameter of from 200 µm to 900 µm and a depth of from 200 µm to 1,000 µm.
Item 3. The method according to Item 1 or 2, wherein the plurality of depressed portions each have an aperture having an approximately circular shape.
Item 4. The method according to any one of Items 1 to 3, wherein the iPS cells are human iPS cells or mouse iPS cells.
Item 5. The method according to Item 4, wherein a culture time in the step (1) is from 0.625 day to 3.5 days.
Item 6. The method according to any one of Items 1 to 5, wherein the culture in the step (2) is performed in the presence of at least one kind selected from the group consisting of a Wnt signal activator and a Hedgehog signal inhibitor.
Item 7. The method according to Item 6, wherein the Wnt signal activator is at least one kind selected from the group consisting of CHIR99021, 6-bromoindirubin-3'-oxime, kenpaullone, SB-216763, SKL2001, deoxycholic acid, WAY-316606, NSC-693868, ricinine, 7-oxo-β-sitosterol, IM-12, HLY78, and retinoic acid.
Item 8. The method according to Item 6 or 7, wherein the Hedgehog signal inhibitor is at least one kind selected from the group consisting of cyclopamine, AY9944, GANT58, GANT61, jervine, SANT-1, SANT-2, U18666A, veratramine, vismodegib, Cur-61414, robotnikinin, JK184, and HPI-4.
Item 9. The method according to any one of Items 1 to 8, wherein the culture in the step (3) is performed in the presence of at least one kind selected from the group consisting of a hypoxia-mimetic compound, a statin compound, and retinoic acid.
Item 10. The method according to any one of Items 1 to 9, further including, before the step (1), a step of culturing the undifferentiated iPS cells without using feeder cells.
Item 11. The method according to any one of Items 1 to 10, wherein the step (1) is performed by placing a suspension of the undifferentiated iPS cells having a cell concentration of from 1.5×10⁵ cells/ml to 3.5×10⁵ cells/ml in the culture vessel, followed by the culture.
Item 12. The method according to any one of Items 1 to 11,
   wherein the step (2) is performed using a culture vessel having at least one well, and
   wherein the step (3) is performed by placing a culture liquid corresponding to 1 to 10 wells containing the mesodermal cells obtained in the step (2) in a culture vessel, followed by the culture.
Item 13. The method according to any one of Items 1 to 12,
   wherein the culture in the step (1) is performed in the presence of a ROCK inhibitor, and
   wherein the culture in each of the step (2) and the step (3) is performed in the presence of retinoic acid.
Item 14. An osteoblast construct being derived from human iPS cells and having a Feret's diameter of from 1 mm to 4 mm.
Item 15. The osteoblast construct according to Item 14, wherein the osteoblast construct is obtained by the method of any one of Items 1 to 13.

### Advantageous Effects of Invention

According to the present invention, the novel method of producing an osteoblast construct, capable of providing an osteoblast construct having a high bone regeneration ability through use of iPS cells as a source material, can be provided.

### Brief Description of Drawings

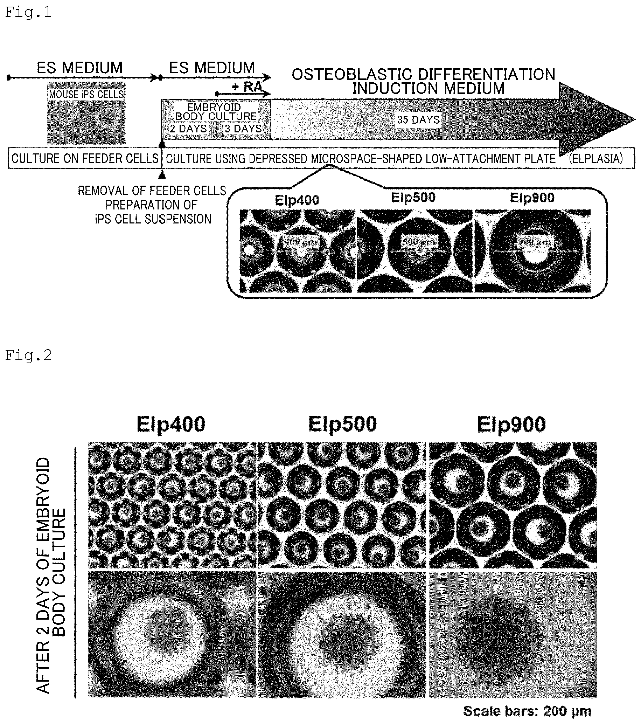

Fig. 1: a method of inducing mouse iPS cells into an osteoblast construct. Through use of a microspace-shaped low-attachment plate Elplasia (trademark) (Kuraray, Japan) having a plurality of depressed portions on the bottom surface of each of its wells, an embryoid body was induced from mouse iPS cells, and an osteoblast construct was generated. Elplasia (trademark) having different depression aperture diameters (400 µm: Elp400, 500 µm: Elp500, and 900 µm: Elp900) were used, and the influence of the difference on the induction into the osteoblast construct was investigated.
Fig. 2 Upper panels: phase-contrast micrographs after mouse iPS cells have been seeded in Elp400, Elp500, and Elp900, respectively, and cultured in an ES medium for 2 days. Lower panels: enlarged photographs of one depressed portion in the Elp400, Elp500, and Elp900, respectively, of the middle panels.

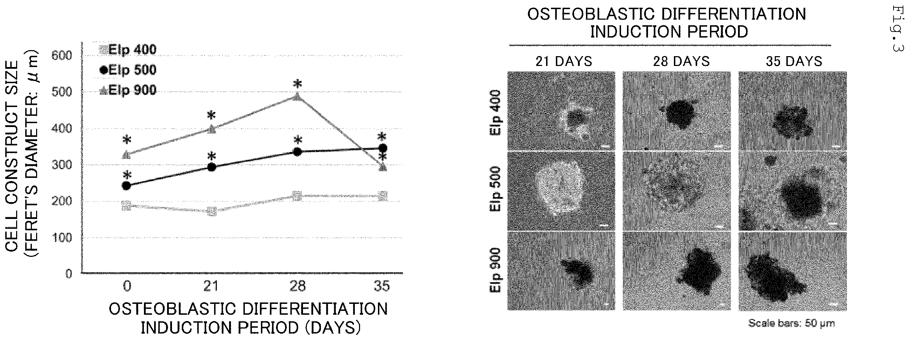

Fig. 3 Left panel: the Feret's diameters of iPS cell constructs in an osteoblastic differentiation induction period (day 0 to day 35) using Elp400, Elp500, and Elp900 (n=3, *P<0.01: ANOVA with Tukey's multiple-comparison test). Right panel: photographs of typical iPS cell constructs in Elp400, Elp500, and Elp900 in the same period.

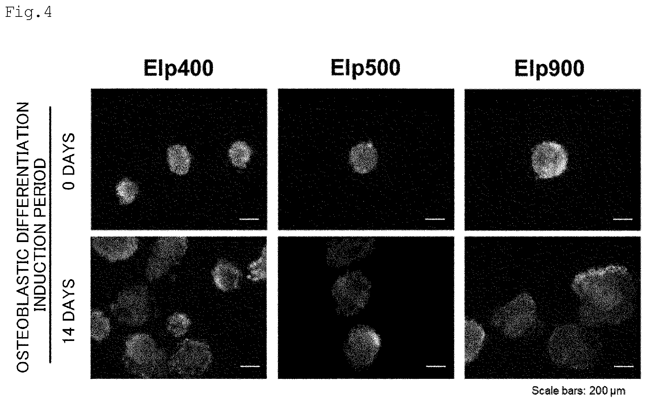

Fig. 4: the survival or death of cells in iPS cell constructs. An investigation was performed using a live cell/dead cell simultaneous staining kit (LIVE/DEAD (trademark) Viability/Cytotoxicity Kit) at the start (day 0) and after 14 days (day 14) of osteoblastic differentiation induction using Elp400, Elp500, and Elp900. Green indicates live cells, and red indicates dead cells.

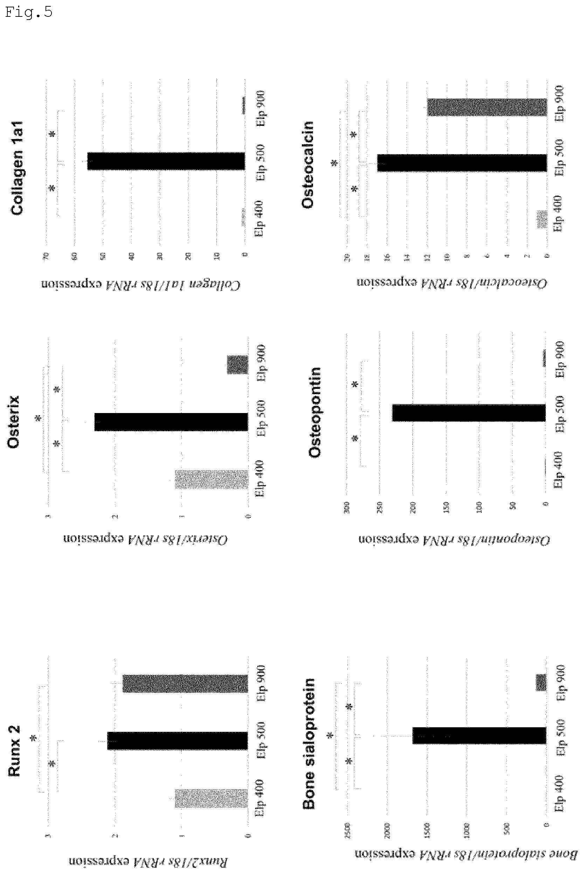

Fig. 5: real-time RT-PCR analysis of the expressions of osteoblast-specific marker genes (Runx2, Osterix, Collagen 1a1, Bonesialoprotein, Osteopontin, and Osteocalcin) in cell constructs after 10 days of osteoblastic differentiation induction using Elp400, Elp500, and Elp900 (n=3, *P<0.05: ANOVA with Tukey's multiple-comparison test).

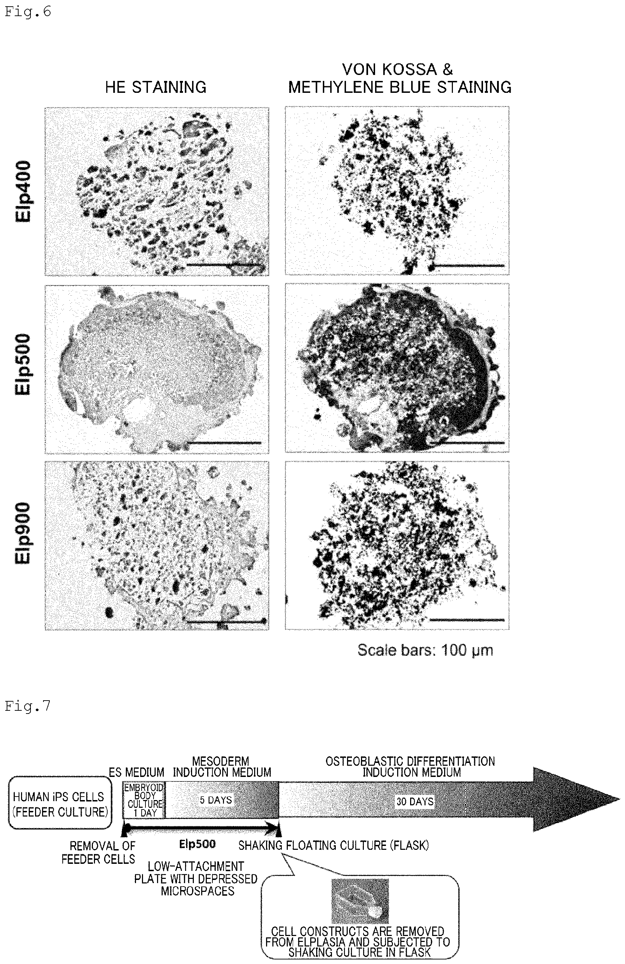

Fig. 6: images of hematoxylin and eosin (HE) staining and double staining with von Kossa and methylene blue of cell construct sections after 35 days of osteoblastic differentiation induction using Elp400, Elp500, and Elp900.
Fig. 7: a method of inducing human iPS cells into an osteoblast construct. Through use of a microspace-shaped low-attachment plate Elplasia (trademark) (Elp500) having a plurality depressed portions each having a diameter of 500 µm on the bottom surface of each of its wells, an embryoid body and mesoderm were induced from human iPS cells, and an osteoblast construct was generated. As a comparative control, a case of using a general low-attachment culture dish instead of using Elplasia (trademark) was set up.

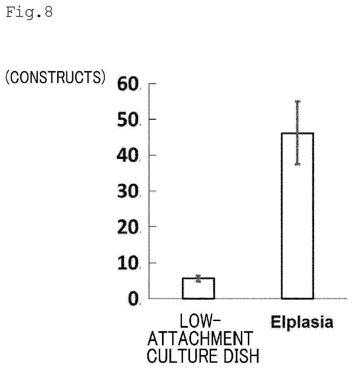

Fig. 8: Human iPS cells were induced into embryoid bodies and mesodermal cells using a low-attachment culture dish or Elp500, and then osteoblastic differentiation induction was performed for 30 days, followed by counting of the numbers of cell constructs in culture flasks. When Elp500 was used, the generation efficiency of osteoblast constructs from human iPS cells was increased about 9-fold (n=5).

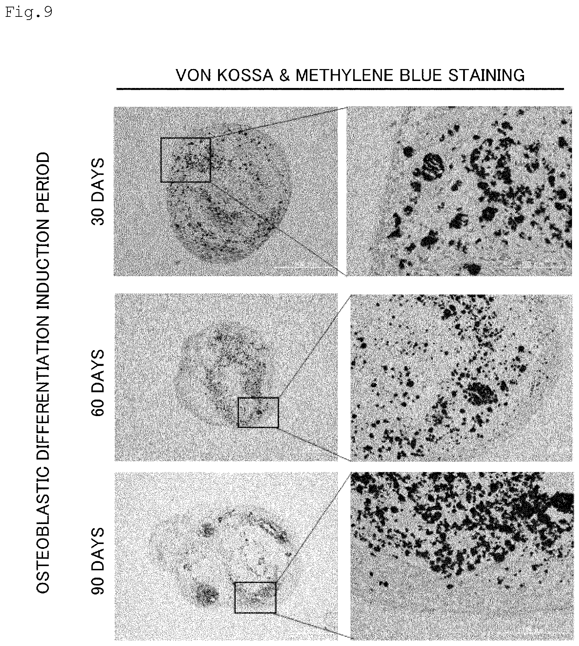

Fig. 9: images of double staining with von Kossa and methylene blue of cell construct sections of human iPS cell constructs subjected to osteoblastic differentiation induction for from 30 days to 90 days after induction into embryoid bodies and mesodermal cells using Elp500.

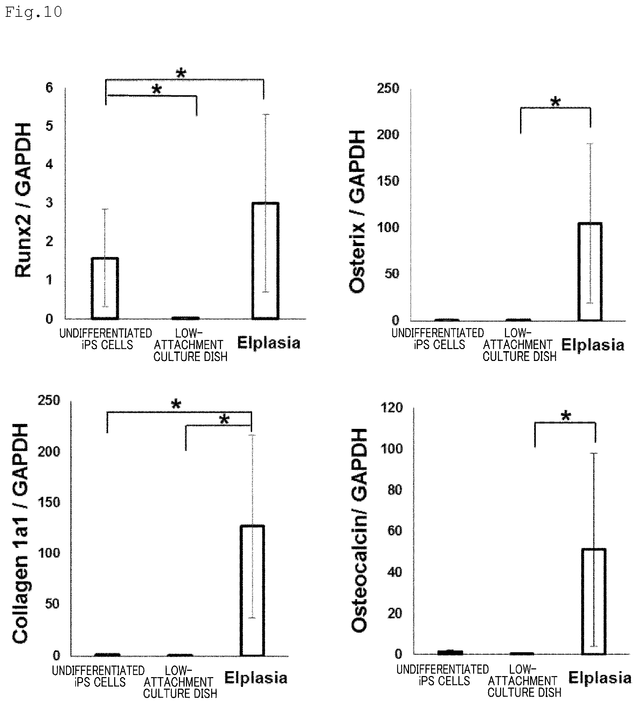

Fig. 10: real-time RT-PCR analysis of the expressions of osteoblast-specific marker genes (Runx2, Osterix, Collagen 1a1, and Osteocalcin) in human iPS cell constructs subjected to osteoblastic differentiation induction for 30 days after induction into embryoid bodies and mesodermal cells using a general low-attachment culture dish (low-attachment culture dish) or Elp500 (Elplasia (trademark)), and undifferentiated human iPS cells immediately before embryoid body culture (undifferentiated iPS cells) (n=3, *P<0.05: ANOVA with Tukey's multiple-comparison test) .

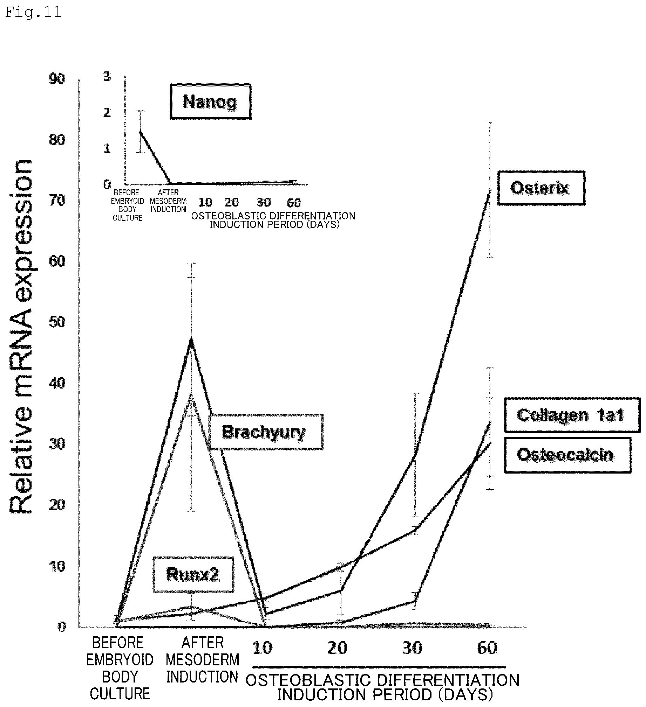

Fig. 11 shows the results of analysis, by a real-time RT-PCR method, of the expressions of an undifferentiation marker gene (Nanog), mesoderm (mesenchymal stem cell)-osteoblast progenitor cell marker genes (Brachyury, Runx2, and Osterix), and osteoblast-specific genes (Collagen 1a1 and Osteocalcin) in human iPS cell constructs subjected to osteoblastic differentiation induction for 60 days after induction into embryoid bodies and mesodermal cells using Elp500, and undifferentiated human iPS cells immediately before embryoid body culture (n=3).

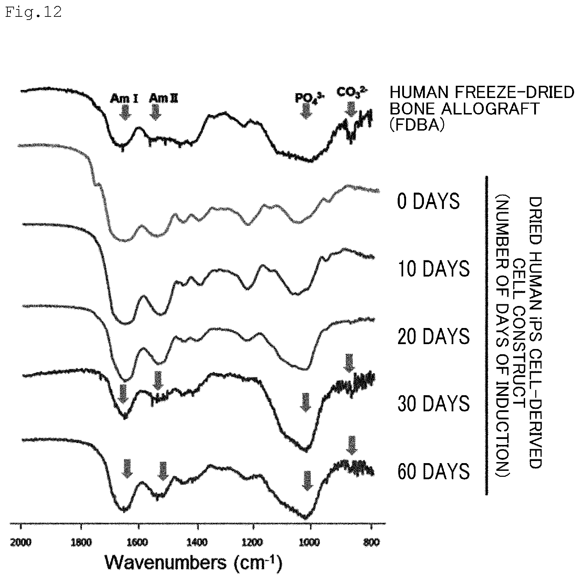

Fig. 12: component analysis of human iPS cell-derived osteoblast constructs using FTIR. Human iPS cell constructs subjected to osteoblastic differentiation induction for from 0 days to 60 days after induction into embryoid bodies and mesodermal cells using Elp500 were dried and then subjected to FTIR analysis. As a comparative control, a human freeze-dried bone allograft (FDBA) was used. Arrows indicate FTIR spectral peaks (Am I, Am II, PO₄³⁻, and CO₃²⁻) that a native bone tissue has.

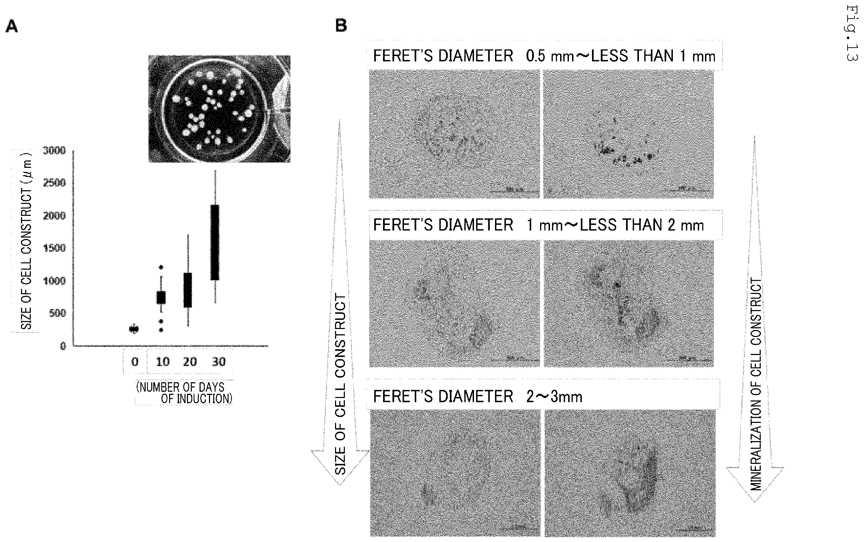

Fig. 13(A): the Feret's diameters of human iPS cell constructs subjected to osteoblastic differentiation induction for from 3 days to 30 days after induction into embryoid bodies and mesodermal cells using Elp500 (n=25). After 30 days of osteoblastic differentiation induction, cell constructs having various sizes ranging from about 0.5 µm to about 3 µm in terms of Feret's diameter were found (insert photograph). Fig. 13(B) shows HE staining images (left column panels) and images of double staining with von Kossa and methylene blue (right column panels) of typical cell construct sections of each of cell construct groups after 30 days of osteoblastic differentiation induction grouped into sizes of from 0.5 mm to less than 1 mm, from 1 mm to less than 2 mm, and from 2 mm to 3 mm in terms of Feret's diameter. As the size of the cell construct increases, more marked mineralization is shown.

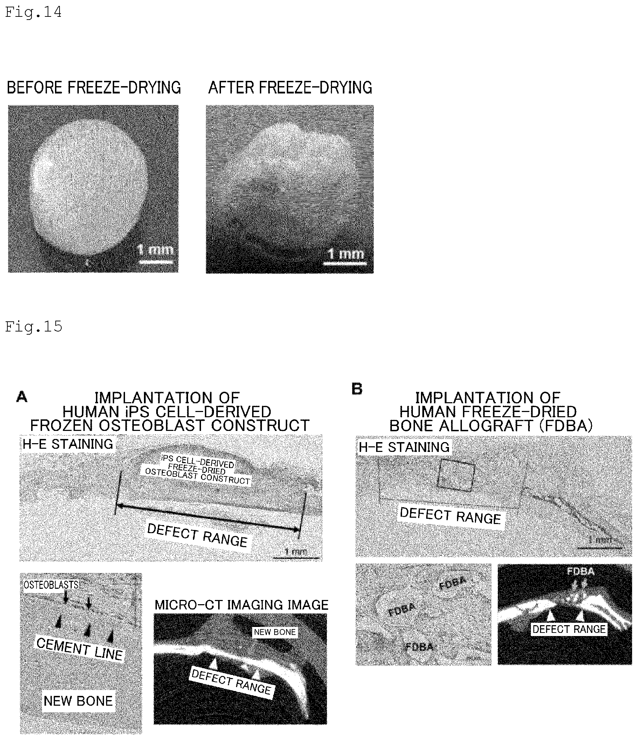

Fig. 14: a photograph (A) of a human iPS cell construct subjected to osteoblastic differentiation induction for 120 days after induction into an embryoid body and mesodermal cells using Elp500 and a photograph (B) after freeze-drying thereof.
Figs. 15: HE staining images and micro-CT imaging images taken 4 weeks after the implantation of (A) the human iPS cell-derived freeze-dried osteoblast construct of Fig. 14 or (B) a human freeze-dried bone allograft (FDBA) into a bone defective site having a diameter of 5 mm generated in the skull of a rat.

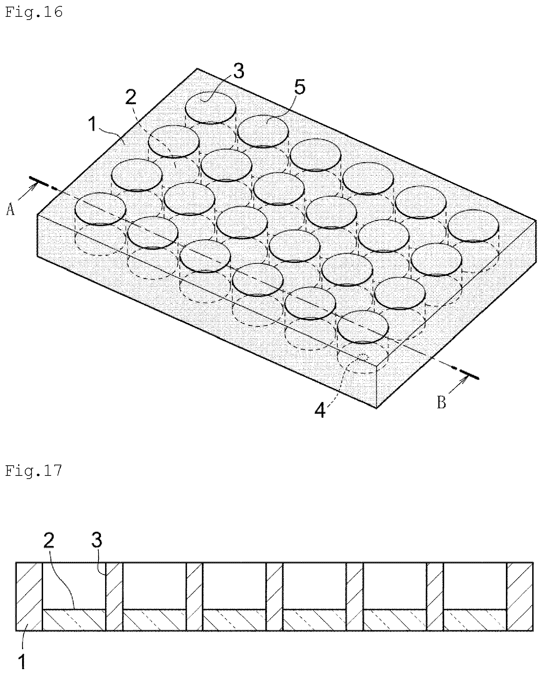

Fig. 16 is an illustration of a microspace culture vessel 1 in a typical embodiment of the present invention.
Fig. 17 is a cross-sectional view of a surface including the dashed line A-B in Fig. 16 and perpendicular to the microspace culture vessel 1.

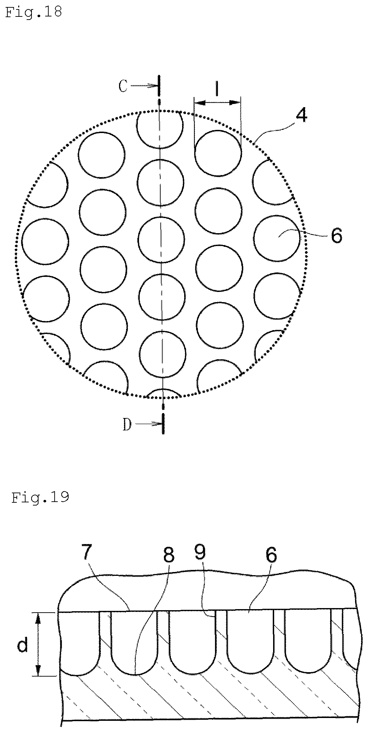

Fig. 18 is an enlarged view of a region 4 in a bottom surface 2 in Fig. 16.
Fig. 19 is a cross-sectional view of the region 4 illustrated in Fig. 18.

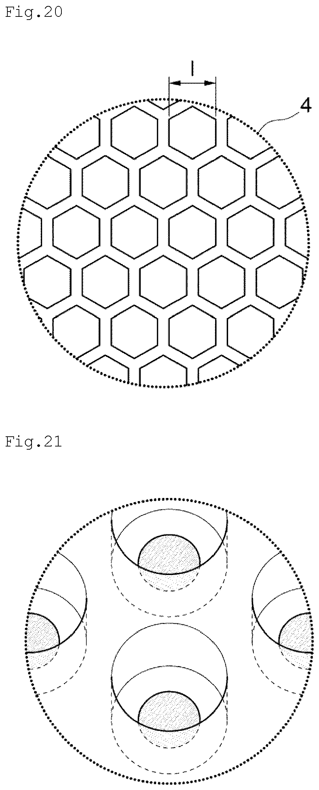

Fig. 20 is an enlarged view of the region 4 in an embodiment in which the shape of each of depressed portions 6 is a regular hexagon.
Fig. 21 is a schematic view for illustrating an embodiment in which one cell construct is accommodated per depressed portion in the microspace culture vessel 1.

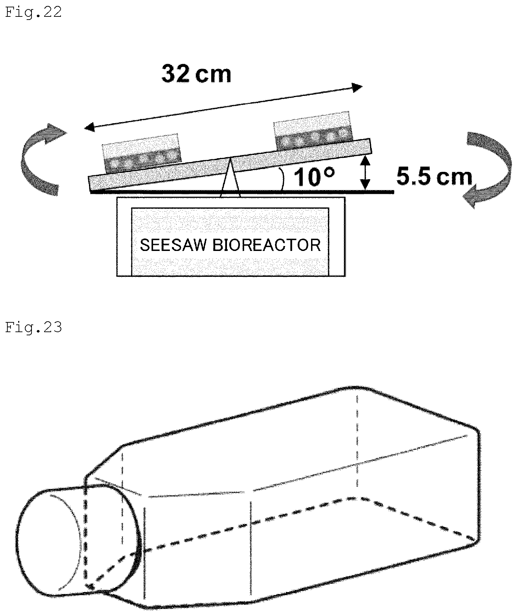

Fig. 22 is an illustration of the outline of a seesaw bioreactor used in Examples.
Fig. 23 is a schematic view of a culture flask that may be used in shaking culture.

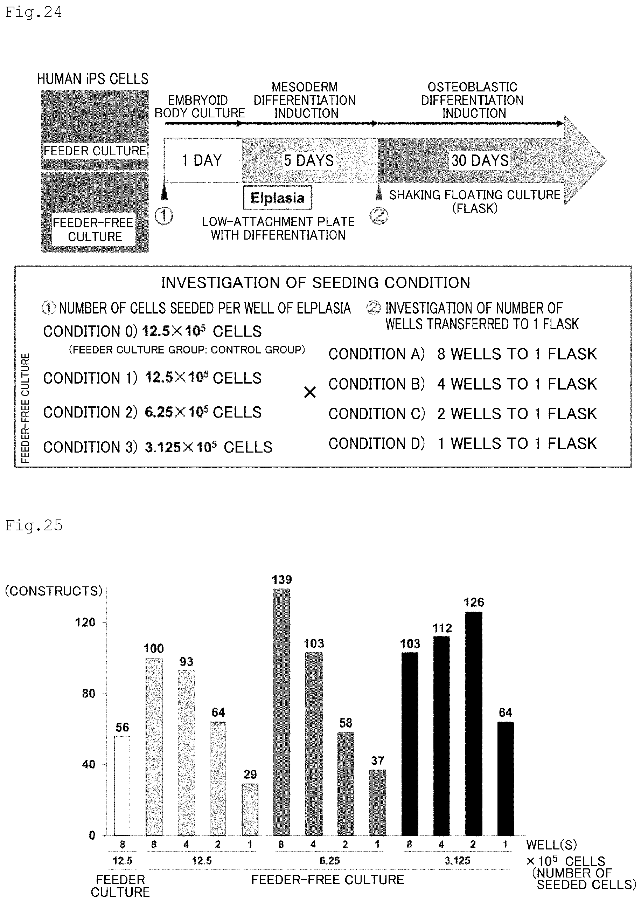

Fig. 24 is an illustration of a method of inducing human iPS cells into an osteoblast construct and condition settings in an investigation of the number of cells used.
Fig. 25 shows the numbers of cell constructs formed per flask on day 30 of osteoblastic differentiation induction. Number of seeded cells; number of cells seeded in 1 well of a 24-well Elplasia (trademark) culture plate in the embryoid body-forming step. Well(s); number of wells of mesoderm cell culture transferred to 1 flask at the start of the osteoblastic differentiation induction step.

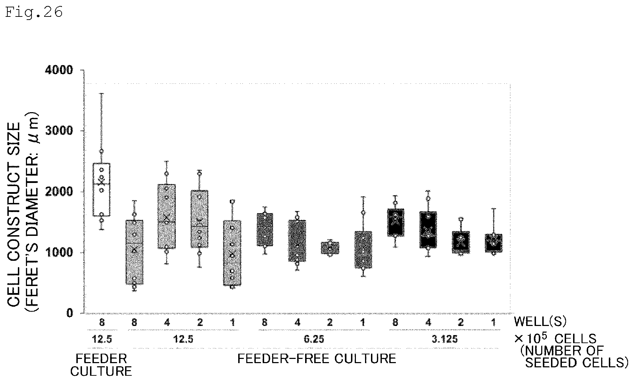

Fig. 26 shows the Feret's diameters of cell constructs on day 30 of osteoblastic differentiation induction (n=10). Number of seeded cells; number of cells seeded in 1 well of a 24-well Elplasia (trademark) culture plate in the embryoid body-forming step. Well(s); number of wells of mesoderm cell culture transferred to 1 flask at the start of the osteoblastic differentiation induction step.

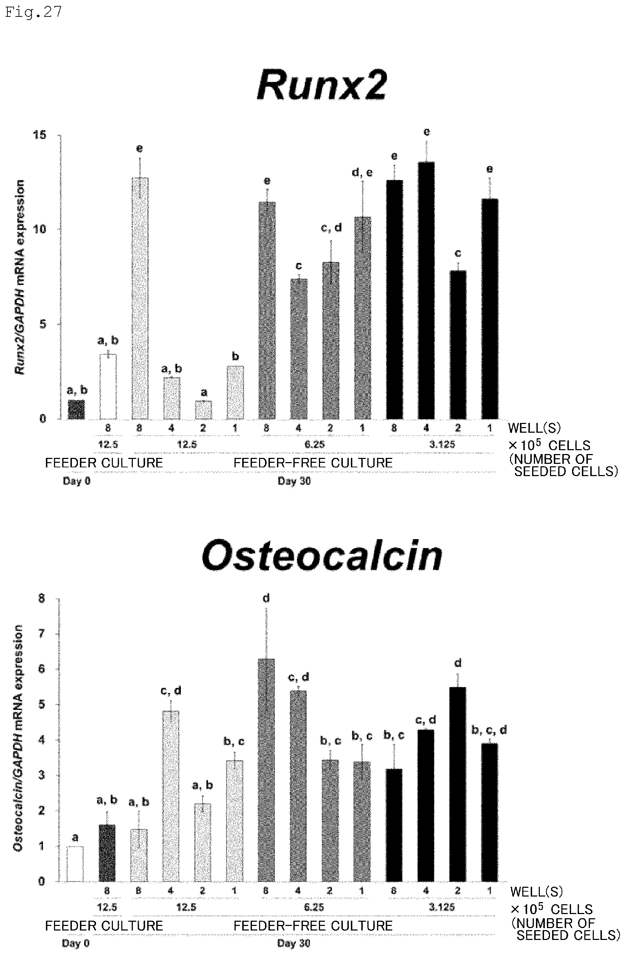

Fig. 27 shows the results of real-time RT-PCR analysis of the expressions of osteoblast-specific marker genes (Runx2 and Osteocalcin) in cell constructs on day 0 and day 30 of osteoblastic differentiation induction (n=3, *P<0.05: ANOVA with Tukey's multiple-comparison test). There is a significant difference between different letters. Number of seeded cells; number of cells seeded in 1 well of a 24-well Elplasia (trademark) culture plate in the embryoid body-forming step. Well(s); number of wells of mesoderm cell culture transferred to 1 flask at the start of the osteoblastic differentiation induction step.

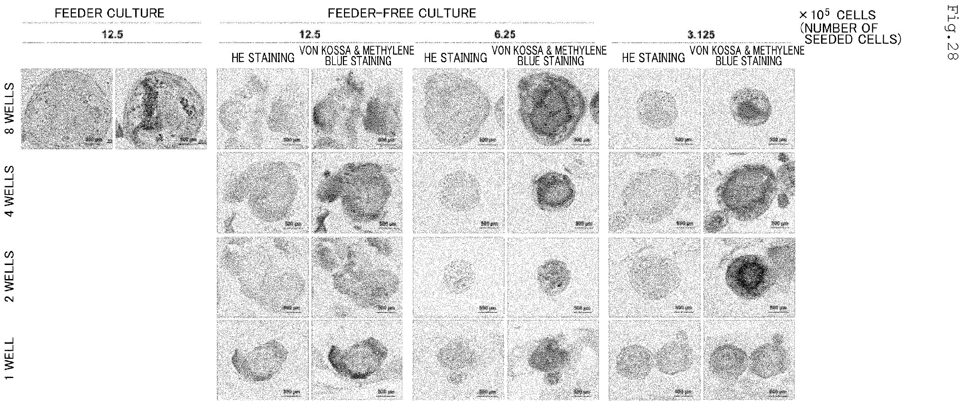

Fig. 28 shows HE staining images (left column panels in each condition) and images of double staining with von Kossa and methylene blue (right column panels in each condition) of cell constructs after 30 days of osteoblastic differentiation induction performed under various conditions. Number of seeded cells; number of cells seeded in 1 well of a 24-well Elplasia (trademark) culture plate in the embryoid body-forming step. 1 well to 8 wells; representing the number of wells of mesoderm cell culture transferred to 1 flask at the start of the osteoblastic differentiation induction step.

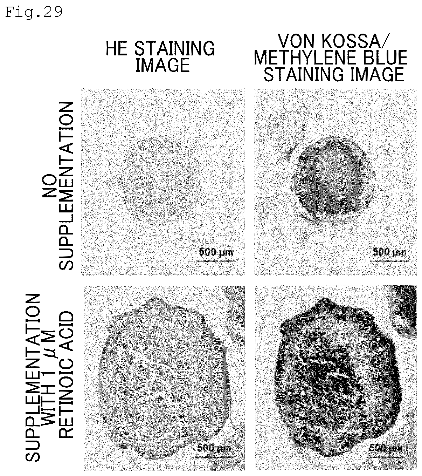

Fig. 29 shows HE staining images (left column panels) and images of double staining with von Kossa and methylene blue (right column panels) of osteoblast constructs in the cases of supplementing and not supplementing a mesoderm differentiation induction medium and an osteoblastic differentiation induction medium with 1 µM retinoic acid in the generation of osteoblast constructs using human iPS cells cultured under a feeder cell-free environment.

### Description of Embodiments

### Method of Producing Osteoblast Construct

The present invention provides a method of producing an osteoblast construct from iPS cells, the method including the steps of:
(1) inducing formation of an embryoid body by subjecting undifferentiated iPS cells to non-adherent culture;
(2) inducing differentiation of the iPS cells into mesodermal cells by subjecting the embryoid body of the iPS cells obtained in the step (1) to non-adherent culture; and
(3) inducing differentiation into osteoblasts by subjecting the mesodermal cells of the iPS cells obtained in the step (2) to non-adherent culture,
wherein the steps (1) and (2) are each performed using a culture vessel comprising a bottom surface and a circular side wall arranged upright on the bottom surface, the bottom surface having a plurality of depressed portions arranged independently of each other.

In the present invention, the "non-adherent culture" means culturing under a state in which the adhesion of cells to the bottom surface and the like (e.g., bottom surface and wall surface) of a culture vessel is suppressed. In the present invention, the non-adherent culture encompasses, for example, shaking culture, and static culture using a non-adherent culture vessel in which the adhesion of cells to the bottom surface and the like of the culture vessel is suppressed (e.g., a non-adherent culture dish, a non-adherent well, a non-adherent flask, a three-dimensional culture plate, or a cell construct generation vessel). As the non-adherent culture vessel, for example, a vessel subjected to low-attachment surface treatment by means of a phospholipid gel, a hydrogel, fine processing, or the like may be used. In addition, in the present invention, for the shaking culture, the above-mentioned non-adherent culture vessel may be used, or a culture vessel that is not non-adherent may be used. When the shaking culture is performed, a method therefor is not particularly limited, but the shaking culture may be performed, for example, using such a seesaw bioreactor as illustrated in Fig. 22. In addition, the inclination angle of the shaking culture is not particularly limited, but is preferably from 1° to 40°, more preferably from 5° to 35°, still more preferably from 10° to 30° against a horizontal direction. The amplitude of the shaking culture is not particularly limited, but may be, for example, from about 0.1 cm to about 20 cm. The cycle of shaking is not particularly limited, but may be, for example, from about 0.01 Hz to about 1.00 Hz. In Fig. 22, the inclination angle is 10°, and the amplitude is 5.5 cm. The width of a table on which a culture flask is to be placed (member of the seesaw bioreactor) is not particularly limited, and a table having a width in the range of, for example, from 20 cm to 50 cm, or from 30 cm to 40 cm may be used. The table culture flask is not particularly limited, but a flask having a culture area (growth area) in the range of, for example, from 5 cm² to 200 cm², from 10 cm² to 60 cm², or from 15 cm² to 35 cm² may be used. In the case where the shaking culture is performed, a cell concentration at the start of the culture is not particularly limited, but when a culture flask having a growth area of 25 cm² is used, the cell concentration is preferably from about 1×10² cells/flask to about 1×10⁸ cells/flask, more preferably from about 1×10⁶ cells/flask to about 7×10⁶ cells/flask.

### Microspace Culture Vessel

The present invention has a feature in that the above-mentioned step of inducing differentiation into germinal cells (step (1)) and the above-mentioned step of inducing differentiation into mesodermal cells (step (2)) are each performed with a culture vessel comprising one or more bottom surfaces and one or more circular side walls arranged upright on the bottom surface, the bottom surface having a plurality of depressed portions arranged independently of each other. In the present invention, the culture vessel including the bottom surface and the circular side wall arranged upright on the bottom surface, the bottom surface having the plurality of depressed portions arranged independently of each other is sometimes referred to as "microspace culture vessel". Similarly, culture using the microspace culture vessel is sometimes referred to as "microspace culture".

A typical embodiment of the present invention is described below with reference to the drawings. First, in Fig. 16, a microspace culture vessel 1 in the typical embodiment of the present invention is illustrated. The microspace culture vessel 1 includes a bottom surface 2 and a circular side wall 3 arranged upright on the bottom surface. Accordingly, the microspace culture vessel 1 may also be said to include wells 5 each formed of the bottom surface 2 and the circular side wall 3 arranged upright on the bottom surface. The circular side wall 3 is not limited to the one having an approximately circular shape at each of the aperture and the bottom surface as illustrated in Fig. 16, and may have, for example, a polygonal shape (a triangular shape, a quadrangular shape (e.g., a rectangular shape, such as a square shape or a rectangle, a parallelogram shape, or a trapezoidal shape)), a pentagonal shape, a hexagonal shape, or the like).

A cross-sectional view of a surface including the dashed line A-B in Fig. 16 and perpendicular to the microspace culture vessel 1 is illustrated in Fig. 17. As in a preferred embodiment illustrated in Fig. 17, a material for forming the wall surface and a material for forming the bottom surface may be different from each other (in the case of Fig. 17, the bottom surface is made of a transparent material, and the wall surface is made of a non-transparent material (e.g., colored black or the like). In addition, an enlarged view of a region 4 in the bottom surface 2 in Fig. 16 is illustrated in Fig. 18. In addition, a cross-sectional view of a surface including the dashed line C-D in the region 4 illustrated in Fig. 18 and perpendicular to an approximate plane formed by the microspace culture vessel 1 is illustrated in Fig. 19. As illustrated in Fig. 18, the bottom surface 2 of the microspace culture vessel 1 in Fig. 16 has a plurality of depressed portions 6 arranged independently of each other. Herein, the depressed portions formed in the bottom surface of the microspace culture vessel (and the wells of the microspace culture vessel) are sometimes referred to as microwells. In the present invention, the shape of each of the depressed portions 6 is not particularly limited as long as the shape has a bottom surface, and examples thereof include ones each having an aperture 7 having an approximately circular shape (e.g., a circular shape as illustrated in Fig. 18) or a polygonal shape (a triangular shape, a quadrangular shape (e.g., a rectangular, such as a square shape or a rectangle, a parallelogram shape, or a trapezoidal shape)), a pentagonal shape, a hexagonal shape, or the like). In the present invention, the "aperture 7" means a portion formed by connecting the highest points of a side wall 9 in one depressed portion 6 (in the case of Fig. 19, the aperture is parallel to the approximate plane formed by the main body of the microspace culture vessel 1). In the present invention, the depressed portions 6 each form a microspace for culturing cells. In the present invention, at least one of the plurality of depressed portions 6 (preferably 80% or more of the number of the depressed portions 6, more preferably 90% or more, typically all) has a circle-equivalent diameter "1" of preferably from 200 µm to 900 µm, more preferably from 400 µm to 700 µm, still more preferably from 450 µm to 550 µm. In the present invention, the "circle-equivalent diameter "1"" means the diameter of the inscribed circle of a plane figure formed by points at each of which a surface parallel to the approximate plane formed by the microspace culture vessel 1 and the side wall 9 intersect each other (for example, the circle-equivalent diameter "1" in the case where the plane figure is a regular hexagon is as illustrated in Fig. 20). When the "plane figure formed by points at each of which a surface parallel to the approximate plane formed by the microspace culture vessel 1 and the side wall 9 intersect each other" varies depending on the depths of the depressed portions, in the present invention, the circle-equivalent diameter "1" refers to the maximum value thereof.

A cross-sectional view of a surface including the dashed line C-D in Fig. 18 and perpendicular to the approximate plane formed by the microspace culture vessel 1 is illustrated in Fig. 19. In the present invention, in each of the depressed portions 6, a bottom surface 8 may have the shape of a curved surface (e.g., a round bottom) as illustrated in Fig. 19, or may be flat. In addition, the side wall 9 may be parallel as illustrated in Fig. 19, or may be tapered (e.g., tapered in such a manner as to be broader on the aperture side and narrower on the bottom surface side). In the present invention, at least one of the plurality of depressed portions 6 (preferably 80% or more of the number of the depressed portions 6, more preferably 90% or more, typically all) has a depth "d" of preferably from 200 µm to 1,000 µm, more preferably from 300 µm to 800 µm, still more preferably from 400 µm to 700 µm. In the present invention, when the surface forming the aperture 7 of the depressed portion and the surface forming the bottom surface 8 thereof are not parallel to each other and/or when the bottom surface is a curved surface, the "depth" of each of the depressed portions 6 refers to the distance to the aperture from the farthest position on the bottom surface of the depressed portion away from the aperture.

In the present invention, the volume of the space formed by each of the depressed portions 6 (space defined by the aperture 7, the bottom surface 8, and the side wall 9) is not particularly limited, but is preferably from 0.02 mm³ to 0.55 mm³, more preferably from 0.04 mm³ to 0.35 mm³, still more preferably from 0.07 mm³ to 0.15 mm³ per depressed portion 6. It is preferred that at least one of the plurality of depressed portions 6 (preferably 80% or more of the number of the depressed portions 6, more preferably 90% or more, typically all) form a space having the above-mentioned volume. In the present invention, the depressed portions preferably have such dimensions as to accommodate one or two (preferably one) iPS cell constructs per each of the depressed portions (e.g., Fig. 21).

The material for forming the microspace culture vessel 1 is not particularly limited, and the vessel is produced from, for example, a resin, such as an acrylic resin, a polystyrene-based resin, an acrylic-styrene-based resin, a polycarbonate-based resin, a melamine resin, polyglycolic acid, polylactic acid, a polyester-based resin, polyimide, a polyvinyl alcohol-based resin, an ethylene-vinyl alcohol-based resin, a thermoplastic elastomer, a vinyl chloride-based resin, or a silicon resin, or a combination thereof.

In order to perform culture while maintaining a state in which the cells or the cell constructs is placed in the depressed portions, culture using the microspace culture vessel 1 is typically performed by static culture. Accordingly, in order to prevent the cells or the cell constructs from adhering to the bottom surface 2 and/or the side wall 3 of the microspace culture vessel 1, in particular, the bottom surface 2, it is preferred that at least part thereof be non-cell-adherent. A method of making the wall surface non-cell-adherent is not particularly limited, but a conceivable example thereof is a method involving applying a non-cell-adherent agent to form a hydrophilic phase on at least part of the wall surface. Examples of the non-cell-adherent agent include agents each containing a hydrophilic polymer, such as polyethylene glycol, a polymer having a betaine structure, a phospholipid-containing polymer, or a polyhydroxyethyl (meth)acrylate polymer.

The present invention has been described above with reference to the drawings, in which a typical embodiment is illustrated, but the present invention is not limited to using the culture vessel illustrated in the drawings. For example, the microspace culture vessel 1 is not limited to such rectangular plate as illustrated in Fig. 16, and may be, for example, a circular plate.

### (1) Induction of Embryoid Body Formation

The method of the present invention includes the step of inducing formation of an embryoid body by subjecting undifferentiated iPS cells to non-adherent culture.

As the iPS cells to be used as a source material, iPS cells generated by introducing a nuclear reprogramming substance into somatic cells may be used. Examples of the iPS cells include iPS cells derived from mammals, such as a human, a mouse, a rat, a monkey, a dog, a pig, a bovine, a cat, a goat, a sheep, a rabbit, a guinea pig, and a hamster. Of those, iPS cells derived from a human, a mouse, a rat, a monkey, a dog, or the like are preferred, and iPS cells derived from a human or a mouse are more preferred.

The somatic cells that may be used as a source material for the generation of the iPS cells may be any cells except germ cells, and examples thereof include oral mucosal cells (e.g., gingival fibroblasts, buccal mucosal fibroblasts, gingival epithelial cells, and buccal mucosal epithelial cells), keratinized epithelial cells (e.g., keratinized epidermal cells), mucosal epithelial cells (e.g., epithelial cells in the lingual epithelium), exocrine gland epithelial cells (e.g., mammary gland cells), hormone-secreting cells (e.g., adrenomedullary cells), metabolizing or storage cells (e.g., hepatocytes), luminal epithelial cells constituting boundary surfaces (e.g., type I alveolar cells), luminal epithelial cells in the closed circulatory system (e.g., vascular endothelial cells), cells with motile cilia (e.g., airway epithelial cells), extracellular matrix-secreting cells (e.g., fibroblasts), contractile cells (e.g., smooth muscle cells), hematopoietic and immune cells (e.g., T lymphocytes), sensory cells (e.g., rod cells), autonomic neurons (e.g., cholinergic neurons), supporting cells for sensory organs and peripheral neurons (e.g., satellite cells), neuronal cells and glial cells in the central nervous system (e.g., astroglial cells), pigment cells (e.g., retinal pigment epithelial cells), and progenitor cells thereof (e.g., tissue progenitor cells). The level of differentiation of the cells is not particularly limited, and both undifferentiated progenitor cells (including somatic stem cells) and terminally differentiated mature cells may be similarly used as a source for the somatic cells in the present invention. Herein, examples of the undifferentiated progenitor cells include tissue stem cells (somatic stem cells), such as neural stem cells, hematopoietic stem cells, mesenchymal stem cells, and dental pulp stem cells.

In the present invention, the "nuclear reprogramming substance" may include any substance (set of substances) capable of inducing iPS cells from somatic cells, such as a protein factor or a nucleic acid encoding the protein factor (including a form of being incorporated into a vector), or a low-molecular-weight compound. When the nuclear reprogramming substance is a protein factor or a nucleic acid encoding the protein factor, preferred examples thereof include the following combinations (only the names of protein factors are given in the following description).
[1] Oct3/4, Klf4, and c-Myc
[2] Oct3/4, Klf4, c-Myc, and Sox2 (herein, Sox2 may be replaced with Sox1, Sox3, Sox15, Sox17, or Sox18. In addition, Klf4 may be replaced with Klf1, Klf2, or Klf5. Further, c-Myc may be replaced with T58A (active mutant), N-Myc, or L-Myc.)
[3] Oct3/4, Klf4, c-Myc, Sox2, Fbx15, Nanog, Eras, ECAT15-2, TclI, and β-catenin (active mutant S33Y)
[4] Oct3/4, Klf4, c-Myc, Sox2, TERT, and SV40 Large T antigen (hereinafter referred to as SV40LT)
[5] Oct3/4, Klf4, c-Myc, Sox2, TERT, and HPV16 E6
[6] Oct3/4, Klf4, c-Myc, Sox2, TERT, and HPV16 E7
[7] Oct3/4, Klf4, c-Myc, Sox2, TERT, HPV6 E6, and HPV16 E7
[8] Oct3/4, Klf4, c-Myc, Sox2, TERT, and Bmil (see WO 2007/069666 A1 for the above-mentioned combinations (provided that, in the above-mentioned combination [2), see Nature Biotechnology, 26, 101-106 (2008) for the replacement of Sox2 with Sox18 or the replacement of Klf4 with Klf1 or Klf5). See also Cell, 126, 663-676 (2006), Cell, 131, 861-872 (2007), and the like for the combination of "Oct3/4, Klf4, c-Myc, and Sox2". See also Nat. Cell Biol., 11, 197-203 (2009) for the combination of "Oct3/4, Klf2 (or Klf5), c-Myc, and Sox2". See also Nature, 451, 141-146 (2008) for the combination of "Oct3/4, Klf4, c-Myc, Sox2, hTERT, and SV40LT".)
[9] Oct3/4, Klf4, and Sox2 (see Nature Biotechnology, 26, 101-106 (2008))
[10] Oct3/4, Sox2, Nanog, and Lin28 (see Science, 318, 1917-1920 (2007))
[11] Oct3/4, Sox2, Nanog, Lin28, hTERT, and SV40LT (see Stem Cells, 26, 1998-2005 (2008))
[12] Oct3/4, Klf4, c-Myc, Sox2, Nanog, and Lin28 (see Cell Research (2008) 600-603)
[13] Oct3/4, Klf4, c-Myc, Sox2, and SV40LT (see also Stem Cells, 26, 1998-2005 (2008))
[14] Oct3/4 and Klf4 (see Nature 454: 646-650 (2008), Cell Stem Cell, 2: 525-528 (2008)))
[15] Oct3/4 and c-Myc (see Nature 454: 646-650 (2008))
[16] Oct3/4 and Sox2 (see Nature, 451, 141-146 (2008), WO 2008/118820 A2)
[17] Oct3/4, Sox2, and Nanog (see WO 2008/118820 A2)
[18] Oct3/4, Sox2, and Lin28 (see WO 2008/118820 A2)
[19] Oct3/4, Sox2, c-Myc, and Esrrb (herein, Essrrb may be replaced with Esrrg. See Nat. Cell Biol., 11, 197-203 (2009))
[20] Oct3/4, Sox2, and Esrrb (see Nat. Cell Biol., 11, 197-203 (2009))
[21] Oct3/4, Klf4, and L-Myc
[22] Oct3/4 and Nanog
[23] Oct3/4
[24] Oct3/4, Klf4, c-Myc, Sox2, Nanog, Lin28, and SV40LT (see Science, 324: 797-801 (2009))
[25] Oct3/4, Klf4, Sox2, and GLIS family members (there are given, for example, GLIS1, GLIS2, and GLIS3, and there is suitably given GLIS family zinc finger 1 (GLIS1). See WO 2010/098419 A1 and WO 2011/102531 A2)
[26] Oct3/4, Klf4, Sox2, and IRX family members (there are given, for example, IRX1, IRX2, IRX3, IRX4, IRX5, and IRX6, and there is suitably given iroquois homeobox protein 6 (IRX6). See WO 2010/098419 A1)
[27] Oct3/4, Klf4, Sox2, and PTX family members (there are given, for example, PITX1, PITX2, and PITX3, and there is suitably given paired-like homeodomain transcription factor 2 (PITX2). Three isoforms (isoforms a, b, and c) are known as PITX2 and any of the isoforms may be used, and isoform b is particularly preferred. See WO 2010/098419 A1)
[28] Oct3/4, Klf4, Sox2, and DMRT-like family B with proline-rich C-terminal 1 (DMRTB1, see WO 2010/098419 A1)

In the above-mentioned combinations [1] to [28], any other Oct family member, such as OctlA or Oct6, may be used instead of Oct3/4. In addition, any other Sox family member, such as Sox7, may be used instead of Sox2 (or Sox1, Sox3, Sox15, Sox17, or Sox18). Further, any other Lin family member, such as Lin28b, may be used instead of Lin28.

In addition, combinations which are not exactly the same as any one of the above-mentioned combinations [1] to [28] but contain all the components in any one of the above-mentioned combinations [1] to [28] and further contain any other substance may also be included in the category of the "nuclear reprogramming substance" in the present invention. In addition, under such a condition that some of the components in any one of the above-mentioned combinations [1] to [28] are endogenously expressed in the somatic cells to be subjected to nuclear reprogramming at a level sufficient for nuclear reprogramming, a combination of only the components other than the above-mentioned components may also be included in the category of the "nuclear reprogramming substance" in the present invention.

Of those combinations, a preferred example of the nuclear reprogramming substance is at least one, preferably two or more, more preferably three or more selected from Oct3/4, Sox2, Klf4, c-Myc, Nanog, Lin28, and SV40LT.

Human cDNA sequence information on the above-mentioned nuclear reprogramming substances may be acquired with reference to NCBI accession numbers described in WO 2007/069666 A1 or WO 2010/098419 A1 (Nanog is described under the name "ECAT4" in these publications. Human cDNA sequence information on Lin28, Lin28b, Esrrb, Esrrg, and L-Myc may be acquired with reference to respective NCBI accession numbers shown in Table 1 below.). A person skilled in the art could easily isolate cDNAs thereof.

**Table 1**

| Gene names | NCBI accession numbers |
|---|---|
| Lin28 | NM_024674 |
| Lin28b | NM_001004317 |
| Esrrb | NM_004452 |
| Esrrg | NM_001438 |
| L-Myc | NM_001033081 |

In addition, human cDNA sequence information on GLIS family members, IRX family members, PTX family members, and DMRTB1 may be acquired with reference to respective NCBI accession numbers shown in Table 2 below.

**Table 2**

| Gene names | NCBI accession numbers | |
|---|---|---|
| | cDNA | Protein |
| IRX1 | NM_010573 | NP_034703 |
| IRX2 | NM_010574 | NP_034704 |
| IRX3 | NM_008393 | NP_032419 |
| IRX4 | NM_018885 | NP_061373 |
| IRX5 | NM_018826 | NP_061296 |
| IRX6 | NM_022428 | NP_071873 |
| GLIS1 | NM_147221 | NP_671754 |
| GLIS2 | NM_031184 | NP_112461 |
| GLIS3 | NM_175459 | NP_780668 |
| PITX1 | NM_011097 | NP_035227 |
| PITX2 (isoform a) | NM_001042504 | NP_001035969 |
| PITX2 (isoform b) | NM_011098 | NP_035228 |
| PITX2 (isoform c) | NM_001042502 | NP_001035967 |
| PITX3 | NM_008852 | NP_032878 |
| DMRTB1 | XM_205469 | XP_205469 |

In addition, a native or artificial mutant protein which has 90% or more, preferably 95% or more, more preferably 98% or more, particularly preferably 99% or more identity to any of the above-mentioned amino acid sequences, and has a comparable nuclear reprogramming ability as a substitute factor of Klf4 as compared to a wild-type protein, and a nucleic acid encoding the mutant protein may also be utilized as a nuclear reprogramming substance of the present invention in place of Klf4.

When the protein factor itself is used as the nuclear reprogramming substance, the protein factor may be prepared by inserting the obtained cDNA into an appropriate expression vector, introducing the expression vector into host cells, culturing the cells, and collecting a recombinant protein factor from the resultant culture. Meanwhile, when the nucleic acid encoding the protein factor is used as the nuclear reprogramming substance, an expression vector is constructed by inserting the obtained cDNA into a viral vector, a plasmid vector, an episomal vector, or the like, and is subjected to a nuclear reprogramming step.

For the introduction of the nuclear reprogramming substance into the somatic cells, a method used in the technical field to which the present invention belongs, such as the method of Patent Literature 1 (WO 2015/64705 A1), may be appropriately used.

In addition, the iPS cells may be obtained by using a factor, such as C4ORF51, HHLA1, ABHD12B, or ZNF541, as an indicator for differentiation resistance, and selecting a line in which such factor is not significantly expressed (see WO 2013/014929 A1).

In addition, the iPS cells to be used may be obtained by, for example, performing culture with feeder cells that supply soluble factors required for, for example, the survival, proliferation, and undifferentiation maintenance of cells and serve as scaffolds for cell adhesion, and appropriately removing the feeder cells as appropriate before embryoid body formation induction. In another embodiment of the present invention, from the viewpoint that the generation efficiency of osteoblast constructs is promoted (a larger number of osteoblast constructs can be obtained), culture of the iPS cells without using feeder cells is preferred over the above-mentioned method involving using feeder cells.

This step may be performed by subjecting those undifferentiated iPS cells to non-adherent culture in a liquid medium to be used for inducing the formation of an embryoid body, through use of the above-mentioned microspace culture vessel.

As the medium, a medium for culturing primate ES/iPS cells may be appropriately used. In the present invention, a medium for culturing ES cells is sometimes referred to simply as ES medium. Examples of the medium for culturing primate ES/iPS cells include: RCHEMD001 manufactured by ReproCELL Inc.; NutriStem manufactured by Biological Industries; Essential 6 Medium manufactured by ThemoFisher Scientific; StemFit AK02N Medium manufactured by Ajinomoto Co., Inc.; and StemFlex Medium manufactured by ThemoFisher Scientific. Such medium may have blended therein growth factors, such as fibroblast growth factors (FGF). Those growth factors may be used alone or in combination thereof. In addition, the medium may optionally have blended therein an additive that may be used in cell culture of stem cells or the like. Specific examples of such additive include fetal bovine serum, amino acids (e.g., L-glutamine), ROCK inhibitors (e.g., Y-27632), and antibiotics (e.g., penicillin, streptomycin, and amphotericin B). In the present invention, a ROCK inhibitor or the like is preferably used as the additive. Through this step, the undifferentiated iPS cells are first grown into an embryoid body instead of directly culturing undifferentiated iPS cells in a mesodermal cell induction medium as in Non-patent Literature 1, and as a result, cell death in the next step for induction into mesodermal cells can be suppressed. Accordingly, in the method of the present invention, it is intended that the embryoid body formation step serving as the step (1) does not substantially cause mesodermal cell induction, and does not proceed beyond the formation of an embryoid body. Accordingly, a medium free of an inducer into mesodermal cells is typically used as the medium to be used in the step (1).

In a typical embodiment of the present invention, this step is performed by placing and holding a suspension of the undifferentiated iPS cells in the above-mentioned medium in the depressed portions of the culture vessel. The number of the undifferentiated iPS cells in the suspension at the time of the placement thereof in the depressed portions of the culture vessel is not limited, but from the viewpoint of promoting the generation efficiency of osteoblast constructs, for example, the cell concentration is preferably from 0.5×10⁵ cells/ml to 7.5×10⁵ cells/ml, more preferably from 1.5×10⁵ cells/ml to 3.5×10⁵ cells/ml. The preferred cell concentration is typically shown as a value in the case of adding 2 ml of the cell suspension per well as in Experimental Examples. In addition, the number of the undifferentiated iPS cells to be added per well at the time of the placement thereof in the depressed portions of the culture vessel is not limited, but from the viewpoint of promoting the generation efficiency of osteoblast constructs, is for example, preferably from 1×10⁵ cells to 15×10⁵ cells, more preferably from 3×10⁵ cells to 7×10⁵ cells. The above-mentioned preferred number of the undifferentiated iPS cells is typically shown as a value in the case of using a plate of the dimensions of a 24-well microspace-shaped low-attachment plate used in Experimental Example 3 [Elplasia (trademark) (Corning, catalog number 4441: having a microwell size measuring 500 µm in diameter and 400 µm in depth, and having 554 to 580 depressions/well)]. In addition, the number of the undifferentiated iPS cells to be placed per depressed portion at the time of the placement thereof in the depressed portions of the culture vessel is not limited, but from the viewpoint of promoting the generation efficiency of osteoblast constructs, is for example, preferably from 100 cells to 3,000 cells, more preferably from 500 cells to 1,200 cells. A culture time in this step is, for example, preferably from about 0.5 day to about 3.5 days, more preferably from 0.625 day to 2.5 days, still more preferably from 0.875 day to 1.25 days. In addition, the culture time in this step is, for example, preferably from about 12 hours to about 84 hours, more preferably from about 15 hours to about 60 hours, still more preferably from about 21 hours to about 30 hours. A case in which the culture time in this step is set within the above-mentioned range is preferred because a final osteoblast construct can be obtained in a solid shape that is not bag-like. A culture temperature in this step is not particularly limited, and is, for example, preferably from 30°C to 42°C, more preferably from 35°C to 39°C. The culture in this step is preferably performed under an atmosphere of 3% to 10% CO₂. In the present invention, the culture in the step (1) makes use of the microspace culture vessel, and hence this step is typically performed by static culture in order to maintain a state in which the cells or the cell constructs are placed in the wells of the microspace culture vessel.

### (2) Induction of Differentiation into Mesodermal Cells

The method of the present invention includes the step of inducing differentiation of the iPS cells into mesodermal cells by subjecting the embryoid body of the iPS cells obtained in the above-mentioned step (1) to non-adherent culture.

As a medium to be used in this step, a medium suitable for differentiation induction of mesodermal cells may be appropriately used. Examples of such medium include: a DMEM medium manufactured by Nacalai Tesque Inc.; a DMEM/F12 medium manufactured by Thermo Fisher Scientific; a Neurobasal medium manufactured by Thermo Fisher Scientific; an RPMI 1640 medium manufactured by Thermo Fisher Scientific; and a Stemline (trademark) II hematopoietic stem cell growth medium manufactured by Sigma-Aldrich Corporation. Those media may be used alone or in combination thereof.

The medium to be used in this step preferably has blended therein a Wnt signal activator from the viewpoint of promoting the induction of differentiation into mesoderm. The Wnt signal activator is not particularly limited, but examples thereof include CHIR99021, 6-bromoindirubin-3'-oxime (BIO), Kenpaullone, SB-216763, SKL2001, deoxycholic acid, WAY-316606, NSC-693868, ricinine, 7-oxo-β-sitosterol, IM-12, HLY78, and retinoic acid (e.g., all-trans-retinoic acid (manufactured by FUJIFILM Wako Pure Chemical Corporation). Of those, CHIR99021 or the like is preferred. Those Wnt signal activators may be used alone or in combination thereof. In a preferred embodiment of the present invention, it is preferred to use retinoic acid in combination with a Wnt signal activator other than retinoic acid. When retinoic acid is blended, its blending amount is not particularly limited, but is, for example, preferably from 0.01 µM to 10 µM, more preferably from 0.1 µM to 5 µM in terms of final concentration in the medium used in this step.

When the Wnt signal activator is blended, its blending amount is not particularly limited, but is, for example, preferably from 1 µM to 100 µM, more preferably from 10 µM to 50 µM in terms of final concentration in the medium to be used in this step.

The medium to be used in this step preferably has blended therein a Hedgehog signal inhibitor from the viewpoint of promoting the induction of differentiation into mesoderm. The Hedgehog signal inhibitor is not particularly limited, but examples thereof include cyclopamine, AY9944, GANT58, GANT61, jervine, SANT-1, SANT-2, U18666A, veratramine, Vismodegib, Cur-61414, Robotnikinin, JK184, and HPI-4. Of those, cyclopamine or the like is preferred. Those Hedgehog signal inhibitors may be used alone or in combination thereof.

When the Hedgehog signal inhibitor is blended, its blending amount is not particularly limited, but is, for example, preferably from 1 µM to 100 µM, more preferably from 1 µM to 10 µM in terms of final concentration in the medium to be used in this step.

In the present invention, it is preferred to use both the Wnt signal activator and the Hedgehog signal inhibitor.

As described in "Solution to Problem" above, the present invention has been completed by making further improvements on the basis of the following novel finding in order to solve the problem involved in the finding: a cell construct obtained by the method involving culture in an ES medium for 2 days, further culture with the addition of retinoic acid for 2 days, and culture in an osteoblastic differentiation induction medium in accordance with the method described in Patent Literature 1 has a hollow bag-like shape. Accordingly, a method involving a culture step using a medium having blended therein only retinoic acid as a component capable of inducing differentiation into mesoderm after the step of inducing embryoid body formation is excluded from the method of the present invention.

In addition, the medium may optionally have blended therein an additive that may be used in cell culture. Specific examples of such additive include fetal bovine serum, amino acids (e.g., L-glutamine), and antibiotics (e.g., penicillin, streptomycin, and amphotericin B).

In addition, the medium may be supplemented with, for example, a commercially available supplement for cell culture as an additive to be used for cell culture. Examples of such supplement include: N-2 Supplement manufactured by Thermo Fisher Scientific; B-27 Supplement manufactured by Thermo Fisher Scientific; Insulin, Transferrin, Selenium Solution manufactured by Thermo Fisher Scientific; Wnt 3a manufactured by R&D Systems; Activin A manufactured by R&D Systems; and BMP4 manufactured by PeproTech. Those supplements may be used alone or in combination thereof. When the above-mentioned step of inducing differentiation into mesodermal cells is performed between (1) the step of inducing embryoid body formation described above and (3) the step of inducing differentiation into osteoblasts to be described later, a solid cell construct can be obtained particularly even in the case of using human iPS cells as the source material.

The method of the present invention is a method for producing a cell construct of osteoblasts. Accordingly, this mesodermal cell differentiation induction step is also performed by non-adherent culture. In the present invention, the culture in the step (2) makes use of the microspace culture vessel, and hence this step is typically performed by static culture in order to maintain a state in which the cells or the cell constructs are placed in the wells of the microspace culture vessel.

In a typical embodiment of the present invention, this step is performed by placing and holding the above-mentioned medium containing the embryoid body obtained in the step (1) in the depressed portions of the culture vessel. A culture time in this step is, for example, preferably from 0.125 day to 10 days, more preferably from 1 day to 8 days, still more preferably from 3 days to 6 days. In addition, the culture time in this step is, for example, preferably from about 3 hours to about 240 hours, more preferably from about 24 hours to about 192 hours, still more preferably from about 72 hours to about 144 hours. A culture temperature in this step is not particularly limited, and is, for example, preferably from 30°C to 42°C, more preferably 35°C to 39°C. The culture in this step is preferably performed under an atmosphere of 3% to 10% CO₂.

### (3) Induction of Differentiation into Osteoblasts

The method of the present invention includes the step of inducing differentiation into osteoblasts by subjecting the mesodermal cells of the iPS cells obtained in the step (2) to non-adherent culture.

As a medium to be used in this step, a medium suitable for the induction of differentiation into osteoblasts may be appropriately used. Examples of such medium include: a DMEM medium (e.g., sodium pyruvate-free DMEM medium manufactured by Nacalai Tesque); and an αMEM medium (e.g., αMEM medium manufactured by Nacalai Tesque). Those media may be used alone or in combination thereof.

In this step, the medium may have blended therein an osteoblastic differentiation induction-promoting agent or the like. Examples of the osteoblastic differentiation induction-promoting agent include ascorbic acid, β-glycerophosphate, dexamethasone, BMP-2, hydrocortisone hemisuccinate, and retinoic acid (e.g., all-trans-retinoic acid). The ascorbic acid may be ascorbate 2-phosphate or a salt thereof. In addition, as a differentiation inducer, instead of or in addition to dexamethasone, hydrocortisone hemisuccinate may be used. Those osteoblastic differentiation induction-promoting agents may be used alone or in combination thereof.

When ascorbic acid is blended, its blending amount is not particularly limited, but is, for example, preferably from 50 µM to 300 µM, more preferably from 150 µM to 200 µM in terms of final concentration in the medium to be used in this step. When β-glycerophosphate is blended, its blending amount is not particularly limited, but is, for example, preferably from 1 mM to 100 mM, more preferably from 5 mM to 15 mM in terms of final concentration in the medium to be used in this step. When dexamethasone is blended, its blending amount is not particularly limited, but is, for example, preferably from 0.001 µM to 10 µM, more preferably from 0.01 µM to 1.0 µM in terms of final concentration in the medium to be used in this step. When retinoic acid is blended, its blending amount is not particularly limited, but is, for example, preferably from 0.01 µM to 10 µM, more preferably from 0.1 µM to 5 µM in terms of final concentration in the medium used in this step.

From the viewpoint of mineralization of osteoblasts, the medium in this step preferably further has blended therein a hypoxia-mimetic compound. Examples of the hypoxia-mimetic compound include desferrioxamine (DFX) and cobalt chloride (CoCl₂). Those hypoxia-mimetic compounds may be used alone or in combination thereof.

From the viewpoint of mineralization of osteoblasts, the medium in this step preferably further has blended therein a statin compound. Examples of the statin compound include atorvastatin, fluvastatin, simvastatin, lovastatin, pitavastatin, pravastatin, and rosuvastatin. Those statin compounds may be used alone or in combination thereof.

In addition, the medium may optionally have blended therein an additive that may be used in cell culture. Specific examples of such additive include fetal bovine serum, amino acids (e.g., L-glutamine), and antibiotics (e.g., penicillin, streptomycin, and amphotericin B).

The method of the present invention is a method for producing a cell construct of osteoblasts. Accordingly, this mesodermal cell differentiation induction step is also performed by non-adherent culture. Specific modes of the non-adherent culture include the above-mentioned ones. In this step (induction of differentiation into osteoblasts), shaking culture or the like is preferred from the viewpoint of promoting differentiation by mechanical stimulation.

In a typical embodiment of the present invention, this step is performed by placing a suspension of the mesodermal cells of the iPS cells obtained in the step (2) in the above-mentioned medium in a culture vessel, for example, a 25 cm² low-attachment flask (Greiner bio-one, growth area), followed by the culture. In the present invention, the amount of the mesodermal cells of the iPS cells in the medium at the start of the culture of the step (3) is not limited, but from the viewpoint of promoting the generation efficiency of osteoblast constructs, for example, mesodermal cells contained in the culture liquid corresponding to 1 to 10 wells of the culture vessel used for the culture of the step (2) are preferably added per 10 ml of the medium, mesodermal cells corresponding to 1 to 5 wells of the culture vessel are more preferably added. For example, mesodermal cells corresponding to 2 to 4 wells of the culture vessel are more preferably added. The above-mentioned preferred amount of the mesodermal cells of the iPS cells is typically shown as a value in the case of using a plate of the dimensions of a 24-well microspace-shaped low-attachment plate used in Experimental Example 3 [Elplasia (trademark) (Corning, catalog number 4441: having a microwell size measuring 500 µm in diameter and 400 µm in depth, and having 554 to 580 depressions/well)]. In addition, mesodermal cells contained in the culture liquid corresponding to 580 to 5,800 depressed portions of the culture vessel used for the culture of the step (2) are preferably added, mesodermal cells corresponding to 580 to 2,900 depressed portions are more preferably added, and mesodermal cells corresponding to 1,160 to 2,320 depressed portions are more preferably added. For the same reason, the step (3) is suitably performed by transferring a mesodermal cell suspension corresponding to 2 ml to 20 ml, preferably 2 ml to 10 ml, more preferably 4 ml to 8 ml of the suspension of the mesodermal cells obtained using the 24-well culture vessel in the step (2) into the 25 cm² low-attachment flask, and changing the medium to 10 ml of the above-mentioned osteoblastic differentiation induction medium. A culture time in this step is, for example, preferably from about 1 day to about 90 days, more preferably from about 7 days to about 60 days, still more preferably from about 21 days to about 50 days. In addition, the culture time in this step is, for example, preferably from about 24 hours to about 2,160 hours, more preferably from about 168 hours to about 1,440 hours, still more preferably from about 504 hours to about 1,200 hours. A culture temperature in this step is not particularly limited, and is, for example, preferably from 30°C to 42°C, more preferably from 35°C to 39°C. The culture in this step is preferably performed under an atmosphere of 3% to 10% CO₂.

According to the present invention, an osteoblast construct having a high degree of mineralization can be obtained as a result of the above-mentioned configurational feature. Further, as described above, according to the present invention, an osteoblast construct having a high bone regeneration ability can be obtained. Accordingly, for example, when the osteoblast construct obtained in the present invention is used as a source material, appropriately subjected to inactivation treatment, and then implanted into a bone defective site, a new bone can be formed to such an extent as to fill such defective site (void), and hence the present invention is extremely useful. Accordingly, the present invention also provides embodiments such as a method of promoting mineralization of iPS cell-derived osteoblasts (or cell construct), a method of enhancing the bone induction ability of iPS cell-derived osteoblasts (or cell construct), and a method of applying stimulation (spatial environment surrounding cells) for inducing self-assembling into a bone to iPS cells during culture. A culture vessel, a material, and various conditions in each of those methods are similar to those described above.

### Osteoblast Construct

When the method of the present invention is carried out using human iPS cells as the source material, an osteoblast construct having a diameter of from 0.5 mm to 5 mm can be obtained. The osteoblast construct being derived from human iPS cells and having such dimension has not been reported heretofore, and hence is a novel osteoblast construct. In addition, the osteoblast construct having such diameter as described above has a high bone regeneration ability, and hence is extremely useful. The diameter of the osteoblast construct falls within preferably the range of from 0.5 mm to 5 mm, more preferably the range of from 1 mm to 4 mm, still more preferably the range of from 1 mm to 3 mm.

### Method of Producing Bone Regeneration Agent

The present invention also provides a method of producing a bone regeneration agent, including the steps of: producing an osteoblast construct from iPS cells by the above-mentioned method; and subjecting the osteoblast construct to inactivation treatment.

The iPS cells serving as a source material to be used in the step of producing an osteoblast construct, and treatment and the like required in the step of producing an osteoblast construct are as described above.

The method of the present invention includes the step of subjecting the osteoblast construct obtained by the above-mentioned step to inactivation treatment.

A method for the inactivation is not particularly limited, but examples thereof include freeze-drying, heat treatment, high-pressure treatment, acid or alkali solution treatment, high-pressure steam sterilization, radiation sterilization, gas sterilization, and electromagnetic wave treatment. Conditions for the freeze-drying are not particularly limited, and a known method may be used. In addition, for example, preliminary freezing may be performed before the freeze-drying. The temperature of the preliminary freezing is not particularly limited, but is preferably, for example, from about -20°C to about -12°C. A freeze-drying temperature is not particularly limited, but is preferably from about -100°C to about -5°C. In addition, a freeze-drying pressure is not particularly limited, and is, for example, preferably 600 Pa or less, more preferably 50 Pa or less. As specific freeze-drying conditions, there is given, for example, a method involving gradually decreasing the pressure to from 5 Pa to 20 Pa, the decrease starting simultaneously with the start of freeze-drying, at a fixed temperature of -10°C. According to the present invention, a bone regeneration agent having a high degree of mineralization and a high bone regeneration ability can be obtained. In one embodiment, the present invention also provides a bone regeneration agent obtained by the above-mentioned production method.

Now, the present invention is more specifically described by way of Examples and Comparative Example. However, the present invention is not limited thereto.

### Examples

### Experiment 1: Search for Optimal Microspace Size for Inducing iPS Cells into Osteoblast Constructs

### «Methods»

### 1.1. Culture of Mouse iPS Cells

For the experiment, an iPS cell line established from mouse gingival fibroblasts [PLoS ONE, 5 (9): e12743, 2010] was used. The mouse iPS cells were maintained and cultured as iPS cell constructs on SNLP76.7-4 feeder cells, which had been treated with mitomycin C, through use of an ES medium [Dulbecco's modified Eagle's medium (DMEM: containing 4.5 g/L glucose and free of sodium pyruvate; Nacalai Tesque, Kyoto) containing 15% fetal bovine serum (Gibco/Life Technologies, Grand Island, NY, USA), 2 mM L-glutamine (Wako Pure Chemical, Osaka), 1×10⁻⁴ M nonessential amino acids (Life Technologies, Grand Island, NY, USA), 1×10⁻⁴ M 2-mercaptoethanol (Life Technologies, Grand Island, NY, USA), 50 U penicillin, and 50 µg/ml streptomycin (Wako Pure Chemical)].

### 1.2. Generation of Osteoblast Constructs of Mouse iPS Cells Using microspace-shaped Low-attachment Plate

The mouse iPS cell constructs cultured above were dissociated into single cells through treatment with 1 mM EDTA-containing 0.25% trypsin (Wako Pure Chemical), and a cell suspension thereof (3.9×10⁶ cells/ml) adjusted with an ES medium was subjected to the experiment. A method by which osteoblast constructs were generated from the mouse iPS cells in this experiment is illustrated in Fig. 1.

As a culture vessel, a microspace-shaped low-attachment plate Elplasia (trademark) (Kuraray, Japan) having microspaces of different depressed sizes (Fig. 1) on the bottom surface of each of the wells of a 6-well plate was used:
Elp400; Cat. #RB 400 560 NA 6: having a diameter of 400 µm and a depth of 560 µm, Elp500; Cat. #RB 500 700 NA 6: having a diameter of 500 µm and a depth of 700 µm, or Elp900; Cat. #RB 900 700 NA 6: having a diameter of 900 µm and a depth of 700 µm. 2 ml of the above-mentioned cell suspension was seeded in each well, and cultured for 2 days to induce the formation of embryoid bodies (Fig. 2).

After the 2 days of culture, the medium in the wells was changed to an ES medium containing 1 µM all trans retinoic acid (RA; Wako Pure Chemical), followed by further culture for 3 days.

After that, the medium was changed to an osteoblastic differentiation induction medium [α-MEM medium (Nacalai Tesque) containing 15% FBS (Gibco/Life Technologies), 0.1 µM dexamethasone (Sigma-Aldrich, St. Louis, MO, USA), 10 mM β-glycerophosphate (Sigma-Aldrich) and 50 µg/ml ascorbate-2-phosphate (Sigma-Aldrich), 1% antibiotic-antimycotic (100 units/ml penicillin, 100 µg/ml streptomycin, and 250 ng/ml amphotericin B (Gibco/Life Technologies)], and culture was performed for up to 35 days. Half of the medium was changed every 2 days. The time point at which the culture in the osteoblastic differentiation induction medium was started was defined as "day 0 of osteoblastic differentiation induction."

### «Results»

### 1.3. Influence of Microspace Diameter on Sizes of Osteoblast Constructs

Cell construct sizes (Feret's diameters) after 5 days of culture from the addition of the iPS cell suspension to the wells of each microspace size (day 0 of osteoblastic differentiation induction) were calculated using Image J image analysis software. As a result, the samples of the Elp400 group, the Elp500 group, and the Elp900 group cultured in the respective microspace had mean Feret's diameters of about 187.6, about 242.5, and about 328.8 µm, respectively, and the sizes of the Elp500 group and the Elp900 group were significantly larger than that of the Elp400 group. During the 35 days after the transfer to the osteoblastic differentiation induction medium, the sizes of the cell constructs in the Elp400 group and the Elp500 group were gradually increased. Meanwhile, the cell construct size in the Elp900 group was increased until day 28 of induction, but was decreased over the period up to day 35 (Fig. 3: left).

The cell construct of each group in osteoblastic differentiation induction was observed under a phase-contrast microscope, and as a result, the cell constructs of the Elp400 group and the Elp900 group were observed to collapse with their contours gradually becoming less smooth over the period from day 21 to day 28 of the induction. Meanwhile, the cell construct of the Elp500 group was present as a cell construct without collapsing until after 35 days of the induction (Fig. 3: right).

The survival or death of the cells in the cell constructs of each group was investigated using a live cell/dead cell simultaneous staining kit (LIVE/DEAD (trademark) Viability/Cytotoxicity Kit, Molecular Probes/Thermo Fisher Scientific, Eugene, OR, USA). As a result, in the Elp400 group and the Elp900 group after 14 days of osteoblastic differentiation induction, red cells indicating dead cells were found in large numbers. In particular, the central portions of the cell constructs of the Elp900 group showed red, that is, an image in which most of the cells inside the cell constructs were dead was found. Meanwhile, the cell constructs of the Elp500 group were formed of green cells representing live cells from the start of the induction until after 14 days of the induction, and dead cells that were red were hardly found (Fig. 4).

### 1.4. Influence of Microspace Diameter on Osteoblastic Differentiation of Cell Constructs

The expressions of osteoblast-specific marker genes (Runx2, Osterix, Collagen 1a1, Bone sialoprotein, Osteopontin, and Osteocalcin) in the cell constructs of each group after 10 days of osteoblastic differentiation induction were analyzed by a SYBR Green real-time RT-PCR method (Thunderbird (trademark) SYBR (trademark) qPCR Mix, TOYOBO). The base sequences of primers used for the SYBR Green real-time RT-PCR method are as shown below. 18s rRNA was used as an internal control.
Runx2 forward primer: 5'-CGGGCTACCTGCCATCAC-3'
Runx2 reverse primer: 5'-GGCCAGAGGCAGAAGTCAGA-3'
Osterix forward primer: 5'-CTCGTCTGACTGCCTGCCTAG-3'
Osterix reverse primer: 5'-GCGTGGATGCCTGCCTTGTA-3'
Collagen 1a1 forward primer: 5'-TGTCCCAACCCCCAAAGAC-3'
Collagen 1a1 reverse primer: 5'-CCCTCGACTCCTACATCTTCTGA-3'
Osteocalcin forward primer: 5'-CCGGGAGCAGTGTGAGCTTA-3'
Osteocalcin reverse primer: 5'-CCGGGAGCAGTGTGAGCTTA-3'
Osteopontin forward primer: 5'-TCTCCTTGCGCCACAGAATG-3'
Osteopontin reverse primer: 5'-TCCTTAGACTCACCGCTCTT-3'
Bone sialoprotein forward primer: 5'-CGGAGGAGACAACGGAGAAG-3'
Bone sialoprotein reverse primer: 5'-GTAAGTGTCGCCACGAGGCT-3'
18s rRNA forward primer: 5'-GTAACCCGTTGAACCCCATT-3'
18s rRNA reverse primer: 5'-CCATCCAATCGGTAGTAGCG-3'

As a result, the expressions of all of those genes in the Elp500 group were significantly higher than those in the Elp400 group and the Elp900 group (Fig. 5).

Further, a section was generated from the cell construct of each group after 35 days of osteoblastic differentiation induction, and was subjected to HE staining, or double staining with von Kossa and methylene blue to perform histological observation.

As a result of the HE staining, the following images were observed: most of the cells in the Elp400 group and the Elp900 group had lost their nuclei and were dead, and the cell constructs were collapsing. Meanwhile, the image was observed: in the cell construct of the Elp500 group, the formation of a layered cell structure was found, and osteoblasts formed a bone-like tissue in the outer layer. As a result of the double staining with von Kossa and methylene blue, in each of the Elp400 group and the Elp900 group, mineralization stained black was found over the entirety of the cell construct, but a brittle and collapsing image with no cell components found was exhibited, suggesting the possibility that mineralization accompanied with cell death occurred. Meanwhile, in the Elp500 group, a rich extracellular matrix accompanied with mineralization was found in the inner layer of the cell structure, and the formation of a bone-like tissue accompanied with partial mineralization was also found in the outer layer (Fig. 6).

### 1.5. Conclusion of Experiment 1

The above-mentioned results revealed that the use of the microspace-shaped low-attachment plate Elplasia (trademark) (Elp500) having depressions each having a diameter of 500 µm enabled efficient generation of three-dimensional osteoblast constructs from mouse iPS cells. In addition, when Elp400 or Elp900 having depressions each having a diameter of 400 µm or 900 µm was used, it was shown to be difficult to generate osteoblast constructs containing live cells from mouse iPS cells.

### Experiment 2: Influence of Depressed Microspace (Elp500) on Osteoblast Construct Induction of Human iPS Cells

### «Methods»

### 2.1. Culture of Human iPS Cells

For an experiment, a human skin fibroblast-derived iPS cell line (409B2: RIKEN BRC CELL BANK) was used. SNLP76.7-4 cells (provided by Dr. Allan Bradley of the Sanger Institute, UK) were used as feeder cells.

The SNLP76.7-4 feeder cells were seeded in a 10 cm cell culture plate (coated with 0.1% gelatin), and cultured using a DMEM medium (sodium pyruvate-free: Nacalai Tesque)] containing 7% fetal bovine serum (FBS: Japan Bio Serum, Lot # JBS-011501), 2 mM L-glutamine (Thermo Fisher Scientific), 50 U penicillin, and 50 µg/ml streptomycin (Thermo Fisher Scientific). The medium was changed every 2 days. Before the culture of iPS cells, the SNLP76.7-4 feeder cells were treated with 12 µg/ml mitomycin C (Nacalai Tesque) for 2.5 hours, and seeded in a 10 cm cell culture plate (coated with 0.1% gelatin) at a concentration of 1.5×10⁶ cells/dish. iPS cells were seeded on the SNLP76.7-4 feeder cells, and cultured using Primates ES Medium (ES medium: REPROCELL) containing 4 ng/ml human basic FGF (REPROCELL). The medium was changed every day.

A method by which osteoblast constructs were generated from the human iPS cells in this experiment is illustrated in Fig. 7.

### 2.2. Formation of Embryoid Bodies

The iPS cells were washed with phosphate buffered saline (PBS), and treated with 1 ml of a CTK solution (0.25% trypsin, 0.1 mg/ml collagenase IV, 10 mM CaCl2, 20% KSR) at 37°C for 1 minute. After that, the CTK solution was removed by aspiration, and 1 ml of PBS was added. PBS was removed, and only the feeder cells exfoliated from the culture plate were removed by aspiration as much as possible. After that, adherent iPS cells remaining in the culture plate were collected using 4 ml of an ES medium. 2 ml of the cell suspension (number of iPS cells in the suspension: 6.25×10⁵ cells/ml) was transferred to 1 well of a microspace-shaped low-attachment plate Elplasia (trademark) (Elp500: Kuraray, Cat.# RB 500 700 NA24) having depressed portions each having a diameter of 500 µm (554 to 580 depressed portions per well) on the bottom surface of each of the wells of a 24-well plate (number of iPS cells placed per well: 12.5×10⁵ cells), and was cultured for 1 day to induce the formation of embryoid bodies. As a control, a group in which embryoid body culture and mesoderm induction were performed using a low-attachment culture dish (Nunc Non-Treated Multidishes, Thermo Fisher Scientific) instead of using Elp500 was set up.

### 2.3. Induction of Differentiation into Mesodermal Cells

One day after the embryoid body formation, the ES medium was changed to 2 ml of a mesoderm differentiation induction medium [1:1 mixed medium of DMEM/F12 (Thermo Fisher Scientific) containing 2% B-27 Supplement (Thermo Fisher Scientific), 1% N-2 Supplement (Thermo Fisher Scientific), 30 µM CHIR99021 (Wako Pure Chemical Industries), and 5 µM cyclopamine (Enzo Life science), and Neurobasal medium (Thermo Fisher Scientific)], and culture was performed for 5 days. The medium was changed every 2 days.

### 2.4. Induction of Differentiation into Osteoblasts

After the mesoderm differentiation induction, cell constructs corresponding to 8 wells (corresponding to 16 ml of the culture liquid) were removed from Elplasia (trademark), and suspended in 10 ml of an osteoblastic differentiation induction medium [DMEM medium (sodium pyruvate-free: Nacalai Tesque, Kyoto) containing 15% FBS (Thermo Fisher Scientific), 0.1 µM dexamethasone (Sigma Aldrich), 10 mM β-glycerophosphate (Sigma Aldrich), 50 µg/ml ascorbate-2-phosphate (Sigma Aldrich), 100 units/ml penicillin, 100 µg/ml streptomycin, and 250 ng/ml amphotericin B (Thermo Fisher Scientific)]. The cell suspension was subjected to low-adherent culture for up to 30 days using a low-attachment flask (Greiner bio-one, growth area: 25 cm²) while being shaken on a seesaw bioreactor (10° inclination, cycle: 0.33 Hz, table width: 32 cm, amplitude: 5.5 cm) (BC-700: BIO CRAFT). The medium was changed every 7 days.

### 2.5. Evaluation of Induction of Differentiation into Osteoblasts

After the 30 days of osteoblastic differentiation induction, the expressions of osteoblastic differentiation-specific genes (Osterix, Collagen 1a1, Runx2, and Osteocalcin) were analyzed by a SYBR Green real-time RT-PCR method (Thunderbird (trademark) SYBR (trademark) qPCR Mix, TOYOBO).

In addition, the expressions of an undifferentiation marker gene (Nanog), osteoblast progenitor cell marker genes (Brachyury and Runx2), and osteoblast marker genes (Osterix, Collagen 1a1, and Osteocalcin) until after 60 days of osteoblastic differentiation induction were analyzed by the SYBR Green real-time RT-PCR method (Thunderbird (trademark)) SYBR (trademark) qPCR Mix, TOYOBO). The base sequences of primers used for the SYBR Green real-time RT-PCR method are as shown below. In addition, GAPDH was used as an internal control.
Runx2 forward primer: 5'-CAGACCAGCAGCACTCCATA-3'
Runx2 reverse primer: 5'-CAGCGTCAACACCATCATTC-3'
Osterix forward primer: 5'-AAGCTGATCTGGTGGTGCAT-3'
Osterix reverse primer: 5'-GACTCCACAAAGGGCATGAT-3'
Collagen 1a1 forward primer: 5'-GTGCTAAAGGTGCCAATGGT-3'
Collagen 1a1 reverse primer: 5'-CTCCTCGCTTTCCTTCCTCT-3'
Osteocalcin forward primer: 5'-CACTCCTCGCCCTATTGGC-3'
Osteocalcin reverse primer: 5'-CCCTCCTGCTTGGACACAAAG-3'
Nanog forward primer: 5'-AACTGGCCGAAGAATAGCAA-3'
Nanog reverse primer: 5'-TGCACCAGGTCTGAGTGTTC-3'
Brachury forward primer: 5'-CAGTCAGTACCCCAGCCTGT-3'
Brachury reverse primer: 5'-ACTGGCTGTCCACGATGTCT-3'
GAPDH forward primer: 5'-GAAGGTGAAGGTCGGAGTCA-3'
GAPDH reverse primer: 5'-GAAGATGGTGATGGGATTTC-3'

Further, the obtained sample was subjected to HE staining, or double staining with von Kossa and methylene blue to perform histochemical observation.

Components of the iPS cell constructs were analyzed using Fourier Transform-InfraRed (FT-IR) spectroscopic analysis. All the iPS cell constructs were collected, fixed with a 10% neutral buffered formalin solution, washed with distilled water, and then gradually dehydrated with ethanol (30%, 70%, 90%, 100%). The ethanol was replaced with fresh ethanol (100%) again, and then the whole was left to stand still in a dryer at 37°C for 12 hours. The dried cell sample was subjected to FT-IR analysis using a potassium bromide (KBr) plate method. An FT-IR measurement apparatus FT/IR-6300ST (JASCO Corporation) was used for the analysis, and an infrared absorption spectral pattern obtained by 1,000 scans over the range of from 650 cm⁻¹ to 4,000 cm⁻¹ at a resolution of 2 cm⁻¹ was analyzed.

### 2.6. Generation of iPS Cell-derived Bone Substitute Material

Osteoblast constructs after 120 days of osteoblastic differentiation induction were washed with PBS, and then immersed in 10 ml of PBS at 4°C overnight. On the following day, the osteoblast constructs were taken out, transferred to a 6 cm cell culture dish, and preliminarily frozen in a freezer at -80°C overnight. After that, the dish was placed in a freeze dryer (VD-250R; Taitec), and freeze-drying was performed overnight to inactivate the cells. Thus, an iPS cell-derived bone substitute material was obtained. The dish having placed therein the bone substitute material was covered with a lid, hermetically sealed with a seal, and stored in a moisture-proof storage (glass desiccator).

### 2.7. Transplantation into Rat Skull Defect Model

A 10-week-old SD rat (Slc:SD; Japan SLC, Inc.) was put under general anesthesia. After that, the scalp was peeled to form a periosteal flap, and a defect having a diameter of 5 mm across the sagittal suture of the skull was formed. The formation of the skull defect was performed using an engine and trephine bar for animal surgery (Implatex, Tokyo) under running water. The freeze-dried iPS cell construct was implanted into the site of the skull defect and covered with the periosteum, and the scalp was sutured. After that, the rat was kept under specific pathogen-free conditions with ad libitum access to water and contact.

### «Results»

### 2.8. Generation Efficiency of Osteoblast Constructs

The numbers of osteoblast constructs on day 30 of osteoblastic differentiation induction obtained from 80% confluent iPS cells in the 10 cm cell culture plate by the methods described in 2.2. to 2.4. above were calculated. The results were 5.7±0.77 in the case of using the low-attachment culture dish, and 46.2±8.8 in the case of using Elp500, revealing that the generation efficiency was remarkably increased by generating embryoid bodies using Elp500 (Fig. 8).

### 2.9. Evaluation of Three-dimensional Osteoblast

### Constructs Generated Using Elp500

Section specimens were prepared from the human iPS cell constructs subjected to the 30 days of osteoblastic differentiation induction after induction into embryoid bodies and mesodermal cells using Elp500, and were subjected to double staining with von Kossa and methylene blue. As a result of histological observation, after the 30 days of osteoblastic differentiation induction, a partially mineralized image was found inside the cell construct (Fig. 9, upper panels). In addition, when osteoblastic differentiation induction was performed for a period of 60 days or 90 days, the mineralized portion inside the cell construct was increased (Fig. 9, middle panels and lower panels). Meanwhile, no mineralization was found in the human iPS cell construct subjected to 30 days of osteoblastic differentiation induction after induction into an embryoid body and mesodermal cells using the low-attachment culture dish, and a partially mineralized image was finally found after 60 days of osteoblastic differentiation induction. Accordingly, it was revealed that the use of Elp500 was able to shorten the period required for a human iPS cell construct to achieve mineralization.

In addition, the expressions of the osteoblast-specific marker genes Runx2, SP7, Collagen 1a1, and osteocalcin in the cell constructs generated using Elp500 on day 30 of osteoblastic differentiation induction were found to be significantly higher than those in the case of using the low-attachment culture dish (Fig. 10).

The expressions of the undifferentiation marker gene (Nanog), marker genes indicating mesoderm (mesenchymal stem cell) to osteoblast progenitor cells (Brachyury, Runx2, and Osterix), and osteoblast-specific genes (Collagen 1a1 and Osteocalcin) in the process of inducing human iPS cells to differentiate to embryoid bodies, mesoderm, and osteoblasts (Fig. 7) were analyzed by a real-time RT-PCR method (Fig. 11). As a result, the expression of Nanog, which was highly expressed in the iPS cells before embryoid body culture, was markedly decreased by mesoderm induction, and nearly disappeared by day 5 of the induction. The expressions of the Brachyury, Runx2, and Osterix genes were found to show peaks of the expressions over the period from after mesoderm induction to an initial stage of osteoblastic differentiation induction, and then the expressions of the Osterix, Collagen 1a1, and Osteocalcin genes were increased with time until after 60 days of differentiation induction. The human iPS cell constructs subjected to osteoblastic differentiation induction for from 0 days to 60 days after induction into embryoid bodies and mesodermal cells using Elp500 were dried, and then subjected to component analysis using FTIR analysis. As a result, the human iPS cell construct samples subjected to 30 days or more of osteoblastic differentiation induction were found to have FTIR spectral peaks similar to those of a native bone tissue (human freeze-dried bone allograft) (Fig. 12).

The Feret's diameters of the human iPS cell constructs subjected to osteoblastic differentiation induction for from 3 days to 30 days after induction into embryoid bodies and mesodermal cells using Elp500 were measured, and as a result, in the cell construct groups after 30 days of osteoblastic differentiation induction, cell constructs varied in size showing Feret's diameters in the range of from 0.66 mm to 2.68 mm were found (Fig. 13A). Those cell construct groups after 30 days of osteoblastic differentiation induction were grouped into sizes of from 0.5 mm to less than 1 mm, from 1 mm to less than 2 mm, and from 2 mm to 3 mm in terms of Feret's diameter, and sections were generated therefrom and subjected to the evaluation of the degree of mineralization through use of von Kossa staining. As a result, the cell constructs of from 2 mm to 3 mm showed mineralization most markedly (Fig. 13B), suggesting that, as the size of the cell construct increased, a more mature osteoblast construct was formed.

### 2.10. Evaluation of Bone Regeneration Ability of Osteoblast Construct Generated Using Elp500

A cell construct generated using Elp500 on day 120 of osteoblastic differentiation induction was subjected to freeze-drying treatment by the method described in 2.6. (Fig. 14).

The freeze-dried osteoblast construct or a human freeze-dried bone allograft (FDBA) serving as a comparative control was implanted into a bone defective site having a diameter of 5 mm generated in the skull of a rat. After 4 weeks from the implantation of the iPS cell-derived freeze-dried osteoblast construct, a tissue image was observed through HE staining of a tissue section. As a result, the bone defect was filled by the formation of a new bone, and a bone remodeling image accompanied with a cement line was found around the implanted osteoblast construct. In addition, as a result of micro-CT analysis, an image in which the bone defective site was completely filled with a new bone continuous with the existing bone therearound was observed (Fig. 15A).

Meanwhile, in the HE staining image after 4 weeks from the implantation of the FDBA, the FDBA was surrounded by immature fibrous tissue, and the formation of a mature new bone was not found. In addition, also in micro-CT analysis, new bone formation was hardly found at the defective site, and the defect was substantially present as it was (Fig. 15B).

### 2.11. Conclusion

Thus, it was revealed that, in the generation of three-dimensional osteoblast constructs from human iPS cells, the use of depressed microspaces, in particular, Elp500 having a specific depressed size in the process of induction into the embryoid body and the mesodermal cells not only increased the generation efficiency in terms of both time and quantity as compared to the case of using a general low-attachment culture dish, but also was able to induce a more mature osteoblast construct. In addition, of the cell constructs generated by this process, cell constructs sized from 2 mm to 3 mm in terms of Feret's diameter on day 30 of osteoblastic differentiation induction were particularly advanced in mineralization, suggesting that the osteoblast constructs were more mature. Further, the freeze-dried human iPS cell-derived osteoblast constructs showed an excellent bone regeneration ability as compared to the existing bone substitute material (FDBA), suggesting applicability as a bone substitute material for promoting the restoration of a bone defective site.

### Experiment 3: Influence of iPS Cell Culture in Feeder Cell-free Environment on Osteoblast Construct Formation

### «Methods»

### 3.1. Human iPS Cells

For the experiment, a human skin fibroblast-derived iPS cell line (409B2: RIKEN BRC CELL BANK) was used.

### 3.2. Culture of Human iPS Cells on Feeder Cells (Feeder-using Group)

SNLP76.7-4 feeder cells were used as feeder cells by the same culture method as in Experiment 2, and human iPS cells were seeded on those cells after Mitomycin C treatment and cultured using Primates ES Medium (ES medium: REPROCELL) containing 4 ng/ml human basic FGF (REPROCELL). The medium was changed every day.

### 3.3. Human iPS Cell Culture under Feeder Cell-free Environment

Human iPS cell colonies that had been maintained and cultured on the feeder cells were collected in a culture liquid obtained by supplementing StemFit (trademark) AK02N medium (ES medium: Ajinomoto Co., Inc.) with 10 µM Y-27632 (Wako Pure Chemical), and seeded in a 10 cm cell culture plate coated with laminin; iMatrix-511 silk (nippi) at a concentration of 8.0× 10⁴ cells/dish. The next day, the medium was changed to an ES medium, and the cells were maintained and cultured. Thus, feeder cell-free culture was performed. The medium was changed once every 2 days.

### 3.4. Generation of Osteoblast Constructs of Human iPS Cells Using Microspace-shaped Low-attachment Plate

A method by which osteoblast constructs were generated from the human iPS cells in this experiment is illustrated in Fig. 24.

### 3.4.1. Induction of Formation of Embryoid Bodies

As a culture vessel, a 24-well microspace-shaped low-attachment plate Elplasia (trademark) (Corning, Cat # 4441: having a microwell size measuring 500 µm in diameter and 400 µm in depth, and having 554 to 580 depressions/well) was used.

The human iPS cells cultured on the feeder cells were prepared into a cell suspension with an ES medium (REPROCELL) in accordance with the method of Experiment 2 (number of iPS cells in the suspension: 6.25×10⁵ cells/ml), and 2 ml thereof was added per well so that the number of seeded cells per well of the Elplasia (trademark) plate was 12.5×10⁵cells (feeder-using group: condition 0).

The human iPS cell colonies cultured under the feeder cell-free environment were dissociated into single cells through treatment with TrypleSelect (Thermo Fisher Scientific), and a cell suspension thereof was adjusted with an ES medium (StemFit medium) to the following three conditions.

| | |
|---|---|
| Condition 1 | 6.25×10⁵ cells/ml |
| Condition 2 | 3.125×10⁵ cells/ml |
| Condition 3 | 1.563×10⁵ cells/ml |

The cell suspension cultured on the feeder cells (condition 0) and the cell suspensions of the conditions 1 to 3 adjusted under the feeder cell-free environment were added in amounts of 2 ml to each well of the above-mentioned culture plate, and the number of cells per well was set as follows.

| | |
|---|---|
| Condition 0 | 12.5×10⁵ cells/well |
| Condition 1 | 12.5×10⁵ cells/well |
| Condition 2 | 6.25-10⁵ cells/well |
| Condition 3 | 3.125×10⁵ cells/well |

The formation of embryoid bodies was induced by 1 day of culture using an ES medium (REPROCELL) for the condition 0, and an ES medium (StemFit medium) for each of the conditions 1 to 3.

### 3.4.2. Induction of Differentiation into Mesoderm Cells

After the 1 day of embryoid body culture, for each condition, in the same manner as in Experiment 2, the medium was changed to a mesoderm differentiation induction medium [1:1 mixed medium of DMEM/F12 (Thermo Fisher Scientific) and Neurobasal medium (Thermo Fisher Scientific), containing 2% B-27 Supplement (Thermo Fisher Scientific), 1% N-2 Supplement (Thermo Fisher Scientific), 30 µM CHIR99021 (Wako Pure Chemical), and 5 µM cyclopamine (Enzo Life science)], followed by culture for 5 days. Half of the medium was changed once every 2 days.

### 3.4.3. Induction of Differentiation into Osteoblasts

After the 5 days of mesoderm differentiation induction, cell constructs contained in the number of wells shown in the following conditions A to D were removed from the Elplasia (trademark) plate under each condition, and transferred to a low-attachment flask (Greiner bio-one, growth area: 25 cm²) .

Condition A 8 wells/1 flask (corresponding to 16 ml of culture liquid)
Condition B 4 wells/1 flask (corresponding to 8 ml of culture liquid)
Condition C 2 wells/1 flask (corresponding to 4 ml of culture liquid)
Condition D 1 well/1 flask (corresponding to 2 ml of culture liquid)

For the group cultured on the feeder cells (condition 0), cells corresponding to 8 wells of the Elplasia (trademark) plate were transferred to 1 flask (condition 0+condition A).

The medium in each flask was changed to 10 ml of an osteoblastic differentiation induction medium [α-MEM medium (Nacalai Tesque) containing 15% FBS (Gibco/Life Technologies), 0.1 µM dexamethasone (Sigma-Aldrich, St. Louis, MO, USA), 10 mM β-glycerophosphate (Sigma-Aldrich), 50 µg/ml ascorbate-2-phosphate (Sigma-Aldrich), and 1% antibiotic-antimycotic (100 units/ml penicillin, 100 µg/ml streptomycin, and 250 ng/ml amphotericin B], and culture was performed for 30 days with shaking on a seesaw bioreactor (10° inclination, cycle: 0.33 Hz, table width: 32 cm, amplitude: 5.5 cm) (BC-700: BIO CRAFT).

Half of the medium was changed once every 3 days. The time point at which the culture in the osteoblastic differentiation induction medium was started was defined as "day 0 of osteoblastic differentiation induction."

### 3.5. Evaluation of Induction of Differentiation into Osteoblasts

After the 30 days of osteoblastic differentiation induction, the number of cell constructs of the group of each condition was counted, and their sizes (Feret's diameters) were calculated using ImageJ image analysis software ((trademark) NIH) .

In addition, the expressions of osteoblast-specific marker genes (Runx2 and Osteocalcin) in the cell constructs of each group after the 30 days of osteoblastic differentiation induction were analyzed by a SYBR Green real-time RT-PCR method (Thunderbird (trademark) SYBR (trademark) qPCR Mix, TOYOBO). GAPDH was used as an internal control. Primers used were the same as those of Experiment 2.

A section was generated from the cell construct of each group after 30 days of osteoblastic differentiation induction, and was subjected to HE staining, or double staining with von Kossa and methylene blue to perform histochemical observation.

### 3.6. Influence of Supplementation of Induction Medium with Retinoic Acid on Formation of Osteoblast Constructs

In the experimental system using human iPS cells under the feeder cell-free environment, the ES medium (StemFit medium) used at the time of the induction of the formation of embryoid bodies in 3.4.1 above was supplemented with 10 µM Y-27632. In addition, the mesoderm differentiation induction medium in 3.4.2 above and the osteoblastic differentiation induction medium in 3.4.3 were supplemented with 1 µM retinoic acid (Wako Pure Chemical), the formation of osteoblast constructs was induced under the condition 2 and the condition B, and cell constructs after 30 days of induction of differentiation into osteoblasts were evaluated for mineralization and maturity by HE staining, and double staining with von Kossa and methylene blue.

### «Results»

### 3.7. Influences of Feeder Cell-free Culture of iPS Cells and Number of Seeded Cells on Number of Osteoblast Constructs Formed

After the 30 days of osteoblastic differentiation induction, in the feeder culture group (condition 0+condition A), the number of cell constructs formed per flask was 56. On the other hand, in the feeder cell-free culture, the numbers of cell constructs formed per flask were as follows: in the case of a number of seeded cells of 12.5×10⁵cells/well (condition 1), 100 constructs in the 8 wells/1 flask group (condition 1+condition A), and 93 constructs in the 4 wells/1 flask group (condition 1+condition B); in the case of a number of seeded cells of 6.25×10⁵ cells/well (condition 2), 139 constructs in the 8 wells/1 flask group (condition 2+condition A), and 103 constructs in the 4 wells/1 flask group (condition 2+condition B); and in the case of a number of seeded cells of 3.125×10⁵ cells/well (condition 3), 103 constructs in the 8 wells/1 flask group (condition 3+condition A), 112 constructs in the 4 wells/1 flask group (condition 3+condition B), and 126 constructs in the 2 wells/1 flask group (condition 3+condition C). That is, in the feeder cell-free culture groups under those conditions, although the numbers of cells used in the embryoid body-forming step and the osteoblastic differentiation induction step were similar to or smaller than those in the feeder culture group (condition 0+condition A), the numbers of osteoblast constructs formed were markedly increased (Fig. 25).

### 3.8. Influences of Feeder Cell-free Culture of iPS Cells and Number of Seeded Cells on Sizes of Osteoblast Constructs

After the 30 days of osteoblastic differentiation induction, the Feret's diameters of the cell constructs in the feeder culture group tended to be larger than the Feret's diameters in the feeder cell-free culture groups. Meanwhile, the group with a number of seeded cells of 6.25×10⁵ cells/well (condition 2) and the group with a number of seeded cells of 3.125×10⁵ cells/well (condition 3) among the feeder cell-free culture groups had smaller variations in size, and hence were more uniform than the feeder culture group (Fig. 26).

### 3.9. Influences of Feeder Cell-free Culture of iPS Cells and Number of Seeded Cells on Osteoblastic Differentiation of Cell Constructs

After the 30 days of osteoblastic differentiation induction, the expressions of osteoblast-specific marker genes (Runx2 and Osteocalcin) in the cell constructs of the feeder cell-free culture groups tended to be high as compared to the cell constructs of the feeder culture group (condition 0+condition A). In particular, in the groups in which, in the embryoid body-forming step, the numbers of seeded cells that were iPS cells that had been cultured under the feeder cell-free environment were 6.25×10⁵ cells/well (condition 2) and 3.125×10⁵ cells/well (condition 3), marked increases in expressions of those genes were found under all of the conditions A to D in the osteoblastic differentiation induction step (Fig. 27).

### 3.10. Influences of Feeder Cell-free Culture of iPS Cells and Number of Seeded Cells on Mineralization of Osteoblast Constructs

After the 30 days of osteoblastic differentiation induction, as compared to the cell constructs of the feeder culture group (condition 0+condition A), the cell constructs in the feeder cell-free culture with a number of seeded cells of 6.25×10⁵ cells/well (condition 2), i.e., the 8 wells/1 flask group (condition 2+condition A) and the 4 wells/1 flask group (condition 2+condition B), the feeder cell-free culture with a number of seeded cells of 3.125×10⁵ cells/well (condition 3), i.e., the 8 wells/1 flask group (condition 3+condition A), the 4 wells/1 flask group (condition 3+condition B), and the 2 wells/1 flask group (condition 3+condition C) had remarkably larger mineralization ranges thereinside, and showed more mature images as osteoblast constructs (Fig. 28).

### 3.11. Influence of Supplementation of Induction Medium with Retinoic Acid on Formation of Osteoblast Constructs of iPS Cells

In the generation of osteoblast constructs using iPS cells under the feeder cell-free environment, when the ES medium in the embryoid body culture was supplemented with 10 µM Y-27632, and the mesoderm differentiation induction medium and the osteoblastic differentiation induction medium were supplemented with 1 µM retinoic acid, marked mineralization was found in the inside and outer layer of cell constructs after 30 days of osteoblasts induction, and a bone-like tissue that was more mature and uniform was obtained than in the case of no supplementation therewith (Fig. 29).

### 3.12. Conclusion

Osteoblast constructs were able to be efficiently generated from a small number of cells by adopting feeder cell-free culture instead of feeder culture in the step of proliferating human iPS cells. In particular, in the case where 6.25×10⁵ human iPS cells that had been cultured under the feeder cell-free environment were seeded per well in the embryoid body-forming step, and further, the osteoblastic differentiation induction step was performed by transferring the culture liquid containing mesodermal cells corresponding to 8 or 4 wells to one flask and in the case where 3.125×10⁵ human iPS cells that had been cultured under the feeder cell-free environment were seeded per well in the embryoid body-forming step, and further, the osteoblastic differentiation induction step was performed by transferring the culture liquid containing mesodermal cells corresponding to 8, 4, or 2 wells to one flask, larger numbers of osteoblast constructs were able to be obtained, and besides, more mature osteoblast constructs were able to be obtained.

## Claims

1. A method of producing an osteoblast construct from iPS cells, the method comprising the steps of:
(1) inducing formation of an embryoid body by subjecting undifferentiated iPS cells to non-adherent culture;
(2) inducing differentiation of the iPS cells into mesodermal cells by subjecting the embryoid body of the iPS cells obtained in the step (1) to non-adherent culture; and
(3) inducing differentiation into osteoblasts by subjecting the mesodermal cells of the iPS cells obtained in the step (2) to non-adherent culture,
wherein the steps (1) and (2) are each performed using a culture vessel comprising a bottom surface and a circular side wall arranged upright on the bottom surface, the bottom surface having a plurality of depressed portions arranged independently of each other.

2. The method according to claim 1, wherein at least one of the plurality of depressed portions has a circle-equivalent diameter of from 200 µm to 900 µm and a depth of from 200 µm to 1,000 µm.

3. The method according to claim 1, wherein the plurality of depressed portions each have an aperture having an approximately circular shape.

4. The method according to claim 1, wherein the iPS cells are human iPS cells or mouse iPS cells.

5. The method according to claim 4, wherein a culture time in the step (1) is from 0.625 day to 3.5 days.

6. The method according to claim 1, wherein the culture in the step (2) is performed in the presence of at least one kind selected from the group consisting of a Wnt signal activator and a Hedgehog signal inhibitor.

7. The method according to claim 6, wherein the Wnt signal activator is at least one kind selected from the group consisting of CHIR99021, 6-bromoindirubin-3'-oxime, kenpaullone, SB-216763, SKL2001, deoxycholic acid, WAY-316606, NSC-693868, ricinine, 7-oxo-β-sitosterol, IM-12, HLY78, and retinoic acid.

8. The method according to claim 6, wherein the Hedgehog signal inhibitor is at least one kind selected from the group consisting of cyclopamine, AY9944, GANT58, GANT61, jervine, SANT-1, SANT-2, U18666A, veratramine, vismodegib, Cur-61414, robotnikinin, JK184, and HPI-4.

9. The method according to claim 1, wherein the culture in the step (3) is performed in the presence of at least one kind selected from the group consisting of a hypoxia-mimetic compound and a statin compound.

10. The method according to claim 1, further comprising, before the step (1), a step of culturing the undifferentiated iPS cells without using feeder cells.

11. The method according to claim 10, wherein the step (1) is performed by placing a suspension of the undifferentiated iPS cells having a cell concentration of from 1.5×10⁵ cells/ml to 3.5×10⁵ cells/ml in the culture vessel, followed by the culture.

12. The method according to claim 11,
wherein the step (2) is performed using a culture vessel having at least one well, and
wherein the step (3) is performed by placing a culture liquid corresponding to 1 to 10 wells containing the mesodermal cells obtained in the step (2) in a culture vessel, followed by the culture.

13. The method according to claim 1,
wherein the culture in the step (1) is performed in the presence of a ROCK inhibitor, and
wherein the culture in each of the step (2) and the step (3) is performed in the presence of retinoic acid.

14. An osteoblast construct obtained by the method of claim 1, the osteoblast construct being derived from human iPS cells and having a Feret's diameter of from 1 mm to 4 mm.
